(19) Europäisches Patentamt
European Patent Office
Office européen des brevets

(11) EP 4 707 274 A1

(12) EUROPEAN PATENT APPLICATION

(43) Date of publication:
**11.03.2026 Bulletin 2026/11**

(21) Application number: **24382968.6**

(22) Date of filing: **10.09.2024**

(51) International Patent Classification (IPC):
***C07D 401/06*** *(2006.01)* ***A61P 35/00*** *(2006.01)*
***A61K 31/4439*** *(2006.01)*

(52) Cooperative Patent Classification (CPC):
**C07D 401/06; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Universidad Autónoma de Madrid**
  **28049 Madrid (ES)**
- **Fundación Imdea Nanociencia**
  **28049 Madrid (ES)**

(72) Inventors:
- **TORTOSA MANZANARES, Mariola**
  **28049 Madrid (ES)**
- **RIGOTTI, Thomas**
  **28049 Madrid (ES)**
- **SOMOZA CALATRAVA, Álvaro**
  **28049 Madrid (ES)**
- **MILÁN ROIS, Paula**
  **28049 Madrid (ES)**

(74) Representative: **ABG Intellectual Property Law, S.L.**
**Avenida de Burgos, 16D**
**Edificio Euromor**
**28036 Madrid (ES)**

(54) **SP3-ANALOGUES OF AXITINIB AND MEDICAL USES THEREOF**

(57) The present invention describes novel Axitinib sp$^3$-analogues wherein the 1,2-disubstituted aromatic ring of commercial Axitinib is replaced by a 1,5-disubstituted bicyclo[2.1.1]hexane scaffold. The invention also describes pharmaceutical compositions comprising said compounds, and the medical uses thereof. The invention also refers to a method for preparing the Axitinib analogues. The new sp$^3$-analogues, which encompass various stereoisomers, pharmaceutically acceptable salts and solvates, have proven more active than marketed Axitinib against several cancer cell lines.



Processed by Luminess, 75001 PARIS (FR)

## Description

### FIELD OF INVENTION

The present invention relates to the field of drug synthesis and therapy. Particularly, the present invention relates to novel Axitinib analogues, pharmaceutical compositions comprising them, and medical uses thereof, specifically in the treatment of cancer. More particularly, the invention relates to compounds of Formula I described below. The invention also encompasses various stereoisomers, pharmaceutically acceptable salts, and solvates of the compounds of Formula (1).

### BACKGROUND

The benzene ring is a prevalent motif found in many marketed drugs, being one of the building blocks for the assembly of molecules in drug discovery programs. This is essentially due to the availability of efficient and complementary methods for the construction of carbon-carbon bonds involving $C(sp^2)$ atoms, in comparison with the incorporation of saturated analogues. Although in some cases the benzene ring contained in the drug skeleton is essential for the establishment of crucial hydrophobic interactions within the enzyme pockets of biological systems, in other cases, this aromatic scaffold is only acting as a linker that connects different functional group-containing fragments. In this context, the benzene substitution pattern and the corresponding exit vectors play a decisive role in the construction of the overall molecular architecture, determining the specific spatial orientation of the drug, which can lead to an efficient interaction with the corresponding receptors in biological systems. Nevertheless, in the last decades, an increasing interest in the use of small $F(sp^3)$-rich scaffolds in drug discovery programs has fostered chemists towards the development of $sp^3$-hybridized bioisostere two-dimensional aromatic cores. Indeed, the replacement of the flat scaffolds that are routinely employed in the pharmaceutical industry with rigid three-dimensional motifs that have well-defined exit vectors can lead to a modulation of the biological and physicochemical properties of the drug candidate, such as its potency and metabolic stability, besides circumventing patent restrictions.

The replacement of a phenyl ring for a saturated scaffold which mimics its substitution pattern was disclosed for the first time by Pellicciari and coworkers (J. Med. Chem. 1996, vol. 39, 2874-2876) through the introduction of a bicyclo [1.1.1]pentane moiety as an appropriate bioisostere of *para*-substituted arenes (Figure 1a). Since then, different strategies have been developed for the incorporation of similar structures into drug-like molecules, establishing a variety of possible bioisosteres of *para*-substituted phenyl rings (Figure 1b). On the other hand, suitable bridged bicyclic scaffolds that mimic the exit vectors of *ortho*- and meta-substituted phenyl rings have been identified only recently (figure 1b). In this context, Denisenko at el. demonstrated that 1,5-disubstituted bicyclo[2.1.1]hexanes are suitable bioisosteres of ortho-substituted aromatic rings (Denisenko A, et al., Angew. Chem. Int. Ed. 2020, vol. 59, 20515-20521). The $sp^3$-hybridized scaffolds were obtained through a racemic approach and validated as bioisosteres through the determination of their physicochemical properties, X-ray crystallographic analysis, and by the evaluation of two agrochemical derivatives as antifungal compounds. Moreover, several groups have explored novel activation modes and strategies to prepare non-linear bridged skeletons as possible analogues of *ortho*- and *meta*-substituted aromatic rings (Figure 1c). While scaffolds that mimic *para*- and *meta*-substituted benzenes shown in Figure 1 represent non-chiral building blocks, bioisosteres of *ortho*-substituted derivatives are chiral structures, including 1,2-and 1,5-disubstituted bicyclo[2.1.1]hexanes (1,2-BCH and 1,5-BCH, Figure 1b) and 1,2-difunctionalized bicyclo[1.1.1]pentanes (1,2-BCP, Figure 1b). Therefore, their synthesis involves an additional level of complexity since both the diastero- and the enantioselectivity need to be controlled. Recently, Pablo Garrido-Garcia et al. (ChemRxiv, 2023, DOI: 10.26434/chemrxiv-2023-f5ll4) reported the first enantioselective catalytic strategy to obtain enantioenriched 1,5-disubstituted bicyclo[2.1.1]hexanes through a Lewis acid-catalyzed intramolecular crossed [2+2] photocycloaddition. While the developed methodology has enabled the incorporation of enantioenriched $sp^3$-bioisostere-containing compounds into a few marketed drugs to prepare different drug analogues, there is an unknown and vast chemical space that could be further explored for the preparation of highly active bioisostere-containing compounds.

Axitinib is a small molecule containing pyridine and indazole moieties developed by Pfizer and is used in the treatment of advanced renal cell carcinoma. Axitinib targets and inhibits specific vascular endothelial growth factor receptors (VEGFR-1, VEGFR-2, and VEGFR-3) at a molecular level. These receptors play a role in angiogenesis (blood vessel formation) which is crucial for tumor growth. By blocking these receptors, Axitinib disrupts tumor blood supply and hinders its development. While Axitinib derivatives, where specific chemical groups are modified, have been explored (e.g. Na Wei et al., Molecules 2018; 23(4):747; doi: 10.3390/molecules23040747), analogues which are based on bioisostere fragments are scarce. For example, Jin-Xin Zhao et al. (Proc. Natl. Acad. Sci. USA. 2021; 118(28): e2108881118; doi: 10.1073/pnas.2108881118) reported 1,2-difunctionalized bicyclo[1.1.1]pentane (BCP) analogues of biologically active molecules, including Axitinib, however, the most potent Axitinib BCP isostere was 250-fold less active than marketed Axitinib. Thus, the synthesis of highly active Axitinib bioisostere-containing compounds is an unmet challenge and more research should focus on obtaining highly active bioisostere-containing compounds holding potential for clinical trials and

ultimately gaining regulatory approval for treatment.

## BRIEF DESCRIPTION OF THE INVENTION

The inventors herein disclose new Axitinib analogues based on the substitution of a 1,2-disubstituted benzene fragment with a 1,5-disubstituted bicyclo[2.1.1]hexane scaffold. The process leading to such analogues comprises a step of Lewis acid-catalyzed [2+2] photocycloaddition. The Axitinib derivatives may be obtained in both a racemic or enantioselective manner, depending on the enantiodifferentiation or lack thereof, in the [2+2] photocycloaddition step. The 1,5-disubstituted bicyclo[2.1.1]hexane scaffold is a bioisostere for an aromatic moiety, and cancer cell viability studies showed that the use of the new analogues with such moiety holds a tremendous potential for *in vivo* therapy. In some cases the biological activity of the two enantiomers were different, and, in both cases, surprisingly higher than marketed Axitinib. This showcases that the control of the absolute configuration and tridimensionality of the drug analogue may have a large impact on its bioactivity, highlighting the need for stereoselective methods towards the construction of the bicyclo [2.1.1] hexane core.

Thus, in a first aspect the invention relates to a compound of formula **I**:

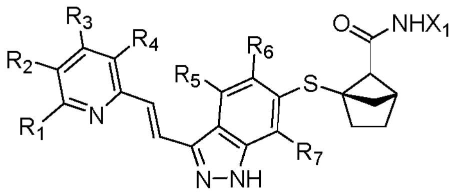

**I** ,

a stereoisomer, a pharmaceutically acceptable salt or a solvate thereof;

wherein $X_1$ is selected from H, C1-C6 alkyl, C1-C6 fluorinated alkyl and C6-C10 aryl; and

wherein groups $R_1$-$R_7$ are independently selected from H, $^2H$, halogen, C1-C6 alkyl, C1-C6 fluorinated alkyl and C6 aryl.

A second aspect relates to a pharmaceutical composition comprising the compound **I** according to the first aspect.

A third aspect relates to a method for preparing a compound of formula **I** as defined above, said method comprising the steps of:

i. reacting a compound of formula **XI** with a compound of formula **XII** under light irradiation and in the presence of a base:

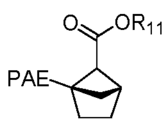

**XI** ,

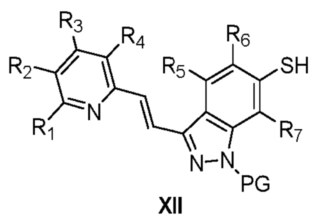

**XII**

to obtain compound **XIII:**

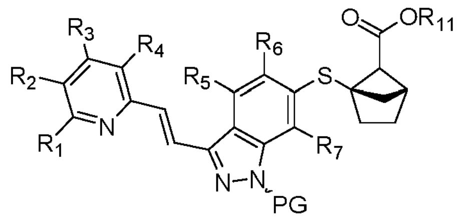

**XIII**

wherein PAE is a photoredox-active ester; PG is a N-protecting group; $R_{11}$ is a C1-C6 alkyl or a C6-C10 aryl; and $R_1$-$R_7$ are independently selected from H, $^2$H, halogen, C1-C6 alkyl, C1-C6 fluorinated alkyl and C6 aryl;

ii. ester hydrolysis of compound **XIII** under basic conditions to obtain free acid **XIV**

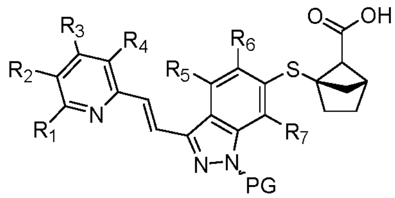

**XIV** ;

iii. reacting free acid **XIV** with an amine of formula $X_1NH_2$ in presence of an acid-activating reagent, wherein $X_1$ is selected from H, C1-C6 alkyl, C1-C6 fluorinated alkyl and C6-C10 aryl, to obtain a compound of formula **XV;**

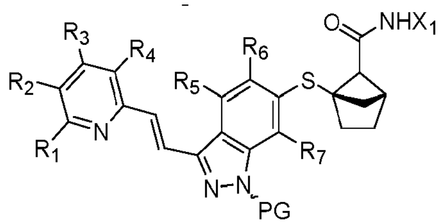

**XV** ;

iv. subjecting compound **XV** to acidic hydrolysis to remove the protecting group (PG) and thereby obtain a compound of formula **I.**

Lastly, the invention relates to the compound of formula **I** according to the first aspect or the pharmaceutical composition according to the second aspect for use in medicine, particularly tin the treatment of cancer.

**BRIEF DESCRIPTION OF THE DRAWINGS**

**Figure 1.** (a) Incorporation of a bicyclo[1.1.1]pentane into a bioactive molecule reported by Pellicciari et al. (b) Bioisosteres of para-disubstituted benzenes and validated or suggested bioisosteres for meta- and ortho-disubstituted benzenes. (c) State-of-the-art strategies for the construction of bridged skeletons as ortho-phenyl bioisosteres and representation of the exit vectors of the 1,5-disubstituted bicyclo[2.1.1]hexane core.

**Figure 2.** Setup for the photochemical reactions.

**Figure 3.** Cell viability studies in different cell lines 72 h after treatment of the two individual sp$^3$-bioisostere-containing enantiomers and the sp$^2$-based marketed Axitinib drug. (A) MB231 cell line. (B) Mel202 cell line. (C) HCT116 cell line.

**Figure 4.** Cell viability studies 72 h after treatment to compare two individual sp$^3$-bioisostere-containing enantiomers

and the $sp^2$-based marketed drugs at the same concentration. (A) *Axitinib* 50 μM in MB231 cell line (B) Axitinib 20 μM in Mel202 cell line. (C) Axitinib 20 μM in HCT116 cell line. Over bar graph represents the X-fold efficacy between enantiomers.

**Figure 5.** Cell cycle and cell death studies. MB231 breast cancer cell line viability studies 72 h after treatment to compare two individual Axitinib $sp^3$-bioisostere-containing enantiomers and the $sp^2$-based marketed Axitinib at 50 μM. Panels A-D: Cell cycle studies. Panels E-H: Cell death studies.

## DETAILED DESCRIPTION OF THE INVENTION

The compounds of the invention are "$sp^3$-bioisoestere-containing compounds" (as they may be called throughout the description in addition to "$sp^3$-drugs" or "$sp^3$-derivatives" or "$sp^3$ analogues") of commercial Axitinib, the chemical structure of which is as follows:

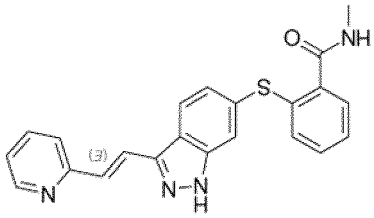

.

As stated above, a first aspect of the invention relates to a compound of formula I:

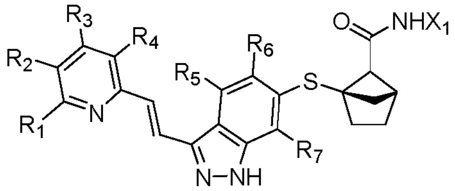

**I**

,

a stereoisomer, a pharmaceutically acceptable salt or a solvate thereof;
wherein $X_1$ is selected from H, C1-C6 alkyl, C1-C6 fluorinated alkyl and C6-C10 aryl; and
wherein groups $R_1$-$R_7$ are independently selected from H, $^2H$, halogen, C1-C6 alkyl, C1-C6 fluorinated alkyl and C6 aryl.

$^2H$ refers to a deuterium substituent. The deuteration can be achieved during the synthesis of compounds of formula I by using appropriate deuterated precursors or by deuteration of the aromatic carbon atoms, e.g. by using deuterium gas ($D_2$) in the presence of a catalyst such as palladium on carbon (Pd/C), platinum (Pt), and rhodium (Rh); heating in presence of $D_2O$ (heavy water), $CD_3OD$ (deuterated methanol), or $C_6D_6$ (deuterated benzene) optionally in the presence of a suitable base or acid; by electrophilic aromatic substitution in presence of DCl, $D_2SO_4$, or $CD_3COOD$; deuterodehalogenation by replacement of halogen atoms (such as Cl, Br, or 1) on the aromatic ring with deuterium from deuterium donor reagents such as $LiAlD_4$ (lithium aluminum deuteride) or $NaBD_4$ (sodium borodeuteride) in a suitable solvent.

The term "C1-C6 alkyl" as used herein refers to a linear or branched alkane derivative containing from 1 to 6 carbon atoms and which is bound to the rest of a molecule through a single bond. Illustrative examples include but are not limited to methyl, ethyl, propyl, butyl, pentyl, hexyl, isopropyl, sec-butyl, tert-butyl, neopentyl, isopentyl, and the like. Included within the definition of'Cl-C6 alkyl" are preferably the groups "C1-C4 alkyl", more preferably the groups "C1-C3 alkyl", even more preferably ethyl and ethyl, most preferably methyl.

The term "C1-C6 fluorinated alkyl" denotes the C1-C6 alkyl groups, as described above, with at least one F atom replacing a hydrogen atom of the alkyl groups. Preferably, the C1-C6 fluorinated alkyl refers to a perfluorinated C1-C6 alkyl, more preferably to a $CF_3$ group.

The term "C6-C10 aryl" refers to an aromatic group having between 6 and 10, preferably 6 or 10 carbon atoms, comprising 1 or 2 aromatic nuclei fused to one another. Illustrative examples of aryl groups include phenyl, naphthyl and indenyl. Preferably, a C6-C10 aryl is a C6 aryl, more preferably a C6 aryl is phenyl. The C6-C10 aryl may be optionally

substituted with a halogen and may encompass also heteroatoms in the aromatic structure (for example, N, S and O), hence being denoted as C6-C10 (hetero)aryl.

The term "halogen" refers to fluorine (F), chlorine (Cl), bromine (Br), iodine (1) substituents.

In a particular embodiment, $X_1$ is selected from:

- H;
- C1-C6 alkyl, preferably C1-C4 alkyl, more preferably C1-C3 alkyl, even more preferably methyl or ethyl;
- C1-C6 fluorinated alkyl, preferably C1-C4 fluorinated alkyl, more preferably C1-C3 fluorinated alkyl, even more preferably fluorinated ethyl or methyl; and
- C6 aryl, preferably phenyl.

In a particular embodiment, $X_1$ is selected from H; C1-C3 alkyl, preferably ethyl or methyl, more preferably $CH_3$; and C1-C3 perfluorinated alkyl, preferably perfluorinated ethyl or methyl, more preferably $CF_3$.

In a preferred embodiment, $X_1$ is selected from H, $CH_3$, ethyl and $CF_3$; most preferably, $X_1$ is $CH_3$.

In an embodiment, groups $R_1$-$R_7$ are independently selected from:

- H;
- $^2$H;
- halogen;
- C1-C6 alkyl, preferably C1-C4 alkyl, more preferably C1-C3 alkyl, even more preferably methyl or ethyl;
- C1-C6 fluorinated alkyl, preferably C1-C4 fluorinated alkyl, more preferably C1-C3 fluorinated alkyl, even more preferably fluorinated ethyl or methyl; and
- C6 aryl, preferably phenyl.

In a particular embodiment of the previous embodiments, the fluorinated alkyl is a perfluorinated alkyl.

In a particular embodiment, $R_1$-$R_7$ are independently selected from H; $^2$H; halogen; C1-C3 alkyl, preferably ethyl or methyl, more preferably $CH_3$; and C1-C3 perfluorinated alkyl, preferably perfluorinated ethyl or methyl, more preferably $CF_3$.

In a preferred embodiment, $R_1$-$R_7$ are independently selected from H, $^2$H, halogen, $CH_3$ or $CF_3$.

In an embodiment, $R_1$-$R_4$ are independently selected from H, $^2$H, halogen, $CH_3$ or $CF_3$.

In an embodiment, $R_5$-$R_7$ are independently selected from H, $^2$H, halogen, $CH_3$ or $CF_3$.

In an embodiment, $R_1$-$R_4$ are H. In another embodiment, $R_5$-$R_7$ are H.

In an embodiment, $R_1$-$R_4$ are independently selected from H, $^2$H, halogen, $CH_3$ or $CF_3$ and $R_5$-$R_7$ are H or $^2$H.

In an embodiment, $R_5$-$R_7$ are independently selected from H, $^2$H, halogen, $CH_3$ or $CF_3$ and $R_1$-$R_4$ are H or $^2$H.

Most preferably, $R_1$-$R_7$ are H.

In a particular embodiment:

- $X_1$ is selected from H; C1-C3 alkyl, preferably ethyl or methyl, more preferably $CH_3$; and C1-C3 perfluorinated alkyl, preferably perfluorinated ethyl or methyl, more preferably $CF_3$; and
- $R_1$-$R_7$ are independently selected from H; $^2$H, halogen; C1-C3 alkyl, preferably ethyl or methyl, more preferably $CH_3$; and C1-C3 perfluorinated alkyl, preferably perfluorinated ethyl or methyl, more preferably $CF_3$.

In another particular embodiment:

- $X_1$ is H, $CH_3$ or $CF_3$, preferably $CH_3$;
- $R_1$-$R_4$ are independently selected from H, $^2$H, halogen, $CH_3$ or $CF_3$, preferably H, $^2$H or halogen, more preferably H; and

- $R_5$-$R_7$ are H.

In a most preferred embodiment:

- $X_1$ is $CH_3$; and
- $R_1$-$R_7$ are independently selected from H, $^2H$ or halogen, preferably H.

In an embodiment, the compound of formula I relates to a mixture of the following stereoisomers:

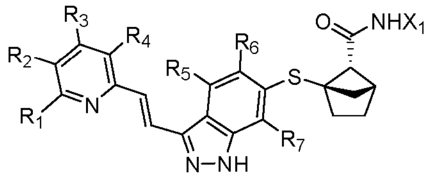

**Ia**

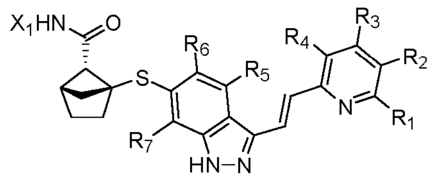

**Ib**

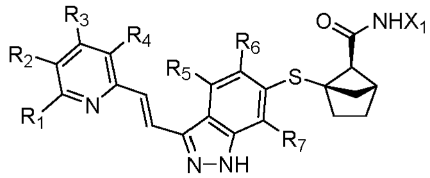

**Ic**

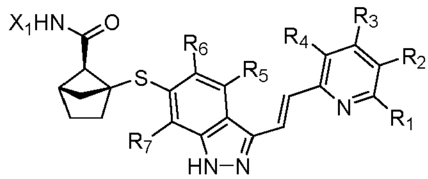

**Id**

,

wherein any of the above embodiments regarding the substituents $R_1$-$R_7$ and $X_1$ applies to the stereoisomers **Ia-Id.**

The mixture of stereoisomers **Ia-Id** can be in any ratio. Preferably, the molar ratio between **Ia+Ib** and **Ic+Id** is comprised between 90:10 and 100:0, more preferably between 95:5 and 100:0; even more preferably **Ia+Ib** is equal to or more than 99 mol% in the mixture of four stereoisomers **Ia-Id.**

In a preferred embodiment, the compound of formula I relates to a mixture of enantiomers **Ia** and **Ib:**

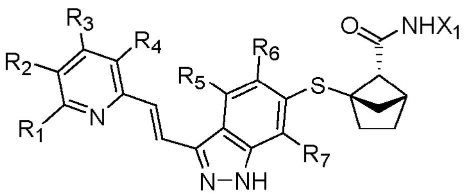

**Ia**

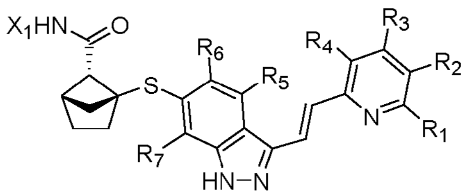

**Ib**

wherein any of the above embodiments regarding the substituents $R_1$-$R_7$ and $X_1$ applies to the enantiomers **Ia** and **Ib.**

The ratio between **Ia** and **Ib** may vary from 99:1 to 1:99. In an embodiment, the ratio between **Ia** and **Ib** is 50:50. In another embodiment, the ratio between **Ia** and **Ib** may vary from 99.9:0.1 to 90:10, preferably from 99: 1 to 95:5, or from 10:90 to 0.1:99.9, preferably from 5:95 to 1:99. More preferably, the ratio between **Ia** and **Ib** is 98:2 or 2:98.

In an embodiment, the compound of formula **I** is compound **Ia.**

In another embodiment, the compound of formula **I** is compound **Ib.**

The pure enantiomers, such as **Ia** or **Ib,** could be obtained according to procedure known to a skilled person in the art, such as resolution, recrystallization, preparative chromatography with a chiral support. By "(enantiomerically) pure" or "pure enantiomer", it is meant that the enantiomer is obtained/detected with an enantiomeric excess (ee) of at least 99.0%, preferably at least 99.5%, more preferably at least 99.9%, meaning that only one enantiomer is observed in large excess with respect to the other enantiomer by techniques such as chiral high-performance liquid chromatography (HPLC), chiral gas chromatography (GC), and polarimetry.

Preferably, the compound of the first aspect is a compound of formula **I:**

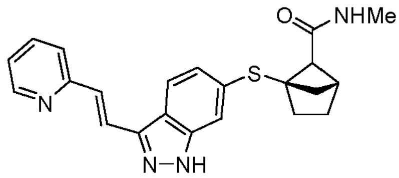

**I**

Preferably, the compound of formula I is a mixture of stereoisomers **Ia'-Id',**

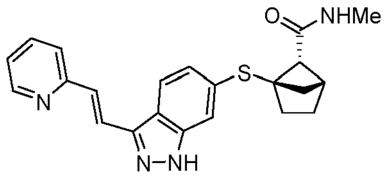

**Ia'**

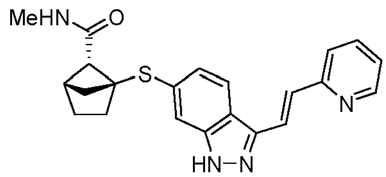

**Ib'**

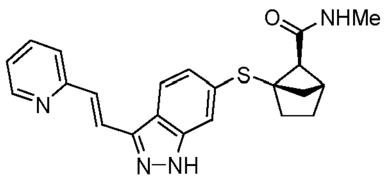

**Ic'**

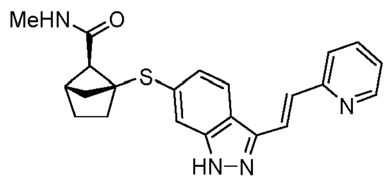

**Id'**

.

The mixture of stereoisomers **Ia-Id** can be in any ratio. Preferably, the molar ratio between **Ia'+Ib'** and **Ic'+Id'** is comprised between 90: 10 and 100:0, more preferably between 95:5 and 100:0; even more preferably **Ia'+Ib'** is equal to or more than 99 mol% in the mixture of four stereoisomers **Ia'-Id'.** The ratio between Ia' and Ib' may be 50:50 or may vary from 99.9:0.1 to 90: 10, preferably from 99: 1 to 95:5, or from 10:90 to 0.1:99.9, preferably from 5:95 to 1:99. More preferably, the ratio between **Ia'** and **Ib'** is 98:2 or 2:98.

More preferably, the compound of formula **I** is a mixture of **Ia'** and **Ib'.** The ratio between **Ia'** and **Ib'** may vary from 99:1 to 1:99. In an embodiment, the ratio between **Ia'** and **Ib'** is 50:50. In another embodiment, the ratio between **Ia'** and **Ib'** may vary from 99.9:0.1 to 90: 10, preferably from 99: 1 to 95:5, or from 10:90 to 0.1:99.9, preferably from 5:95 to 1:99. More preferably, the ratio between **Ia'** and **Ib'** is 98:2 or 2:98.

In an embodiment, the compound of formula **I** is enantiomerically pure **Ia'.**

In another embodiment, the compound of formula **I** is enantiomerically pure **Ib'.**

The compound of formula **I** according to first aspect, or any of the stereoisomers mentioned above, may be in the form of a pharmaceutically acceptable salt or a solvate thereof.

Pharmaceutically acceptable salts refer to ionic compounds formed by the reaction of the compound of formula **I** with a suitable acid. These salts are considered safe for administration to a patient. Converting a drug into a salt can offer several advantages, including improved solubility (salts often dissolve more readily in water or other solvents, which can be crucial for absorption into the bloodstream after administration), enhanced stability (their shelf life and handling characteristics may be improved) and controlled release (by tuning the rate at which the drug releases its active form in the body).

Pharmaceutically acceptable salts of the compounds of formula **I** include, but are not limited to, salts obtained from the addition of hydrochloric acid, hydrobromic acid, sulfuric acid, phosphoric acid, acetic acid, citric acid, tartaric acid. The addition of the above-mentioned acids protonate the $-NHX_1$ group into a $-NH_2X_1$ group with a positive charge counterbalanced by the anion of the acid employed for salt generation.

The compounds of formula **I** may be crystalline or amorphous solids, preferably they are crystalline. A solvate refers to specific crystalline forms where the compound of formula **I** is physically associated with a defined number of solvent molecules. These solvent molecules are incorporated into the crystal structure of the compound of formula **I.** The inclusion of the solvent may happen due to factors like hydrogen bonding or van der Waals interactions between the compound of formula **I** and solvent molecules.

The solvate of the compound of formula **I** may be a monohydrate, a dihydrate or a solvate with n molecules of solvent (n is an integer >2). Typical solvates are formed by inclusion of water or aliphatic alcohols, such as ethanol.

Pharmaceutical compositions

In another aspect, the invention refers to a pharmaceutical composition comprising the compound **I,** preferably a compound of formula **Ia** and/or **Ib,** more preferably a compound of formula **Ia'** and/or **Ib',** according to any of the embodiments of the first aspect.

The pharmaceutical composition of the second aspect further comprises one or more pharmaceutically acceptable excipients to facilitate formulation and enhance drug delivery. The selection and manipulation of type and amount of pharmaceutically acceptable excipients is within the skills of a person with ordinary skills in the art. Typical pharmaceutically acceptable excipients include, but are not limited to, viscosity modifiers, fillers, diluents, lubricants, glidants. disintegrants, antioxidants, pH modifiers, taste-masking agents, and combinations thereof.

Typical viscosity modifiers include hydroxypropyl methylcellulose (HPMC), methylcellulose (MC), sodium carboxymethyl cellulose (NaCMC), ethylcellulose, xanthan gum, guar gum, carrageenan, polyethylene glycol (PEG), poloxamer, carbomer, acrylate polymers (e.g., Carbopol®), bentonite, magnesium aluminum silicate, Veegum®, hydroxyethyl cellulose (HEC), Gellan gum, povidone (PVP), alginates, gelatin, and polyvinyl alcohol (PVA).

Typical fillers include lactose (e.g., lactose monohydrate, lactose anhydrous), microcrystalline cellulose (e.g., Avicel®), dibasic calcium phosphate (e.g., dibasic calcium phosphate dihydrate, dibasic calcium phosphate anhydrous), mannitol, sorbitol, sucrose, starch (e.g., corn starch, potato starch), dextrose, calcium sulfate, talc, sodium chloride, polyvinylpyrrolidone (PVP), silicified microcrystalline cellulose, silica (colloidal silicon dioxide), kaolin, bentonite, magnesium aluminum silicate, glycine, pregelatinized starch, maltodextrin.

Typical diluents include lactose (e.g., lactose monohydrate, lactose anhydrous), microcrystalline cellulose (e.g., Avicel®), dibasic calcium phosphate (e.g., dibasic calcium phosphate dihydrate, dibasic calcium phosphate anhydrous), mannitol, sorbitol, sucrose, starch (e.g., corn starch, potato starch), dextrose, calcium sulfate, talc, sodium chloride, polyvinylpyrrolidone (PVP), silicified microcrystalline cellulose, silica (colloidal silicon dioxide), kaolin, bentonite, magnesium aluminum silicate, glycine, pregelatinized starch, and maltodextrin.

Typical lubricants include magnesium stearate, calcium stearate, sodium stearyl fumarate, stearic acid, talc, polyethylene glycol (PEG), sodium lauryl sulfate, glyceryl behenate, hydrogenated vegetable oil, colloidal silicon dioxide, and povidone (PVP).

Typical glidants include talc, colloidal silicon dioxide (silica), magnesium stearate, calcium stearate, sodium stearyl fumarate, aerosil®, corn starch, and polysorbate 80.

Typical disintegrants include starches (e.g., corn starch, potato starch), croscarmellose sodium, sodium starch glycolate, crospovidone, microcrystalline cellulose, sodium carboxymethyl cellulose (NaCMC), cross-linked Povidone (PVPP), alginic acid, and methylcellulose.

Typical antioxidants include tocopherol, butylated hydroxytoluene (BHT), butylated hydroxyanisole (BHA), ascorbic acid (Vitamin C), propyl gallate, ascorbyl palmitate, alpha-lipoic acid, citric acid, dodium metabisulfite, glutathione, cysteine, rosemary extract, and green tea extract.

Typical pH modifiers include citric acid, tartaric acid, acetic acid, hydrochloric acid, sodium hydroxide, potassium hydroxide, sodium bicarbonate, phosphoric acid, lactic acid, and sodium citrate.

Typical taste-masking agents include saccharin, aspartame, acesulfame potassium, sucralose, cyclodextrins, flavors (e.g., fruit flavors, mint flavors), menthol, ethyl maltol, vanillin, citric acid, tartaric acid, malic acid, sucrose, mannitol, sorbitol, xylitol, isomalt, polyols, and gelatin.

The pharmaceutical composition of the second aspect can be formulated into various dosage forms including tablets, capsules, suspensions, solutions, or injectables, depending on the intended route of administration. The route of administration may be oral, intracutaneous, intravenous, and intramuscular, among others.

The pharmaceutical composition comprises at least 1 wt% of a compound of formula **I,** preferably at least 5 wt%, more preferably at least 10 wt%, even more preferably at least 20 wt%, even more preferably at least 30 wt% of a compound of formula **I.**

Methods of preparation

A third aspect of the invention refers to method for preparing a compound of formula **I** according to the first aspect. Said method of the third aspect comprises the following steps:

i. reacting a compound of formula **XI** with a compound of formula **XII** under light irradiation and in the presence of a base:

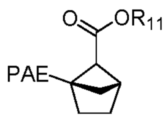 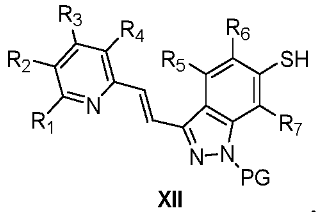

XI , XII ,

to obtain compound XIII:

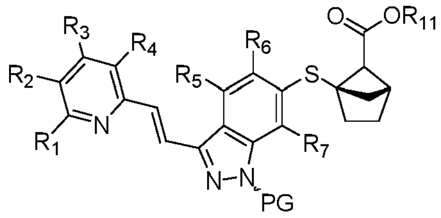

**XIII**

wherein PAE is a photoredox-active ester; PG is a N-protecting group; $R_{11}$ is a C1-C6 alkyl or a C6-C10 aryl; and $R_1$-$R_7$ are independently selected from H, $^2H$, halogen, C1-C6 alkyl, C1-C6 fluorinated alkyl and C6 aryl;

ii. ester hydrolysis of compound **XIII** under basic conditions to obtain free acid **XIV:**

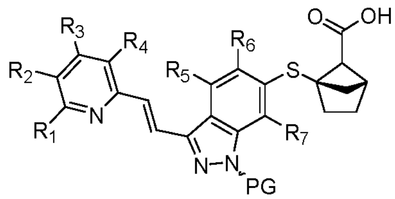

**XIV** ;

iii. reacting free acid **XIV** with an amine of formula $X_1NH_2$ in presence of an acid-activating reagent, wherein $X_1$ is selected from H, C1-C6 alkyl, C1-C6 fluorinated alkyl and C6-C10 aryl, to obtain a compound of formula **XV;**

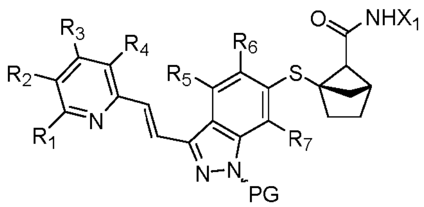

**XV** ;

iv. subjecting compound **XV** to acidic hydrolysis to remove the protecting group (PG) and thereby obtain a compound of formula **I.**

Compound **XI** may be racemic or an enantioenriched mixture of enantiomers. The latter can be obtained according to the method disclosed in the below embodiments and examples.

In step i, a reaction between compound **XI** which contains a photoredox-active ester (PAE) group and thiol **XII** takes place under light irradiation and in the presence of a base. Such reactions involving PAE groups are known in the art (for example in Pablo Garrido-Garcia et al., ChemRxiv, 2023, DOI: 10.26434/chemrxiv-2023-f5ll4, wherein PAE is a phthalimide fragment), thus compounds of formula **XI** were chosen as suitable starting materials for the synthesis of compounds of formula **I.** Compounds of formula **XII** are either commercial or can be readily obtained according to known procedures [see for example L.-H. Zhai et al., Org. Process Res. Dev. 2015, 19, 849-857, wherein 6-nitro indazoles are subjected to iodination, N-protection, Heck coupling with 2-vinyl pyridine derivatives, nitro reduction, Sandmeyer reaction,

(metal-catalyzed, metal-mediated or photochemical) C-S cross-coupling reaction followed by hydrolysis of the thioester to obtain the free thiol (i.e. employing thioacetate salts, followed by hydrolysis of the thioester). The corresponding 6-nitro indazoles can be obtained from a variety of *ortho*-methyl anilines upon nitration via electrophilic aromatic substitution, diazotization and following cyclization (see for example: P. R. Kym, et al., J. Med. Chem. 2006, 49, 7, 2339-2352; B.-L. Li et al., Chinese Chemical Letters 2014, 25, 989-994; R. A. Bartsch et al., J. Heterocycl. Chem. 1984, 21, 1063-1064)]. Alternatively, compounds of formula **XII** can be readily obtained according to known procedures [see for example J. Yu, et al., Beilstein J. Org. Chem. 2018, 14, 786-795, wherein commercially available 6-bromo or 6-iodo indazoles are subjected to a sequence of bromination or iodination, N-protection, Heck coupling with 2-vinyl pyridine derivatives, (metal-catalyzed, metal-mediated or photochemical) C-S cross-coupling reaction followed by hydrolysis of the thioester to obtain the free thiol (i.e. employing thioacetate salts, followed by hydrolysis of the thioester)]. Moreover, compounds of formula **XII** can be derivatized and transformed according to known procedures of aromatic halogenation, deuteration, aromatic electrophilic substitution (alkylation and (per)fluoro alkylation) or catalytic and non-catalytic cross-coupling reactions. PG is a N-protecting group. Such groups are well known in the art; preferably pyrazolyl protecting groups are employed such as tetrahydropyran-2-yl (THP), tert-butoxycarbonyl (Boc), carbobenzyloxy (Cbz), 9-fluorenylmethyloxycarbonyl (Fmoc), trimethylsilyl (TMS), tosyl (Ts), 2-(trimethylsilyl)ethoxymethyl (SEM), benzyl (Bn); preferably the N-protecting group is THP.

The PAE group in compound **XI** may be of different nature, preferably is selected from a N-hydroxyphthalimide (NHPI) ester, a thioester, a benzyl ester and an acyloxime ester, more preferably is a NHPI ester, therefore the formula of compound **XI** would be as shown below:

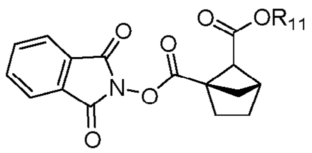

or a derivative thereof wherein the benzene ring is optionally substituted with a halogen, a C1-C6 alkyl, a C1-C6 fluorinated alkyl or a C6-C10 aryl.

Typical syntheses of compounds of formula **XI** are analogous to that reported in the examples of the present application (see for example the synthesis of compound **10**).

In a preferred embodiment, $R_{11}$ is a C1-C6 alkyl, more preferably a C1-C2 alkyl, even more preferably is a methyl group.

Preferred embodiments for groups $R_1$-$R_7$ have been described for the first aspect of the invention.

Upon visible light irradiation, an electron donor-acceptor (EDA) complex, also called charge-transfer complex, is formed between compound **XI** and a deprotonated form of **XII** (obtained upon deprotonation with DIPEA). The EDA complex can be excited to obtain an excited state charge-transfer complex. Eventually, a single-electron transfer takes place between the electron-donor component (deprotonated **XII**) and the electron-acceptor component **XI.** This electron transfer results in the formation of a thiyl radical and the subsequent cleavage of the ester bond of the PAE ester (such as the cleavage of the N-O bond of the NHPI ester of compound **XI**), producing an alkyl radical, $CO_2$ and a leaving group (such as a phthalimide anion). Thus, the thiyl and the alkyl radicals can recombine through a radical coupling to deliver compound **XIII** by formation of a new C-S bond.

Preferably, step i takes place in presence of DIPEA (diisopropylethyl amine), although any organic or inorganic base could be employed (i.e. triethylamine, $Na_2CO_3$, $K_3PO_4$, $Cs_2CO_3$).

The irradiation is preferably performed by blue LED irradiation, more preferably at $\lambda$ = 400-500 nm, even more preferably at $\lambda$ =440 nm.

Step i is preferably carried out in a solvent. The solvent may be any polar organic solvent, such as DMSO, DMF, acetone, acetonitrile, dichloromethane and N,N-Dimethylacetamide. Preferably, the solvent is DMSO.

The reaction time of step i is at least 5 min, at least 10 min, at least 20 min, at least 30 min, at least 1 hour; preferably is comprised between 5 min and 10 hours, more preferably between 1 hour and 4 hours, even more preferably is about 2 hours.

Product **XIII** is obtained as a mixture of diastereoisomers, preferably in a 1:1 molar ratio.

In step ii, ester hydrolysis of compound **XIII** is performed under basic conditions to obtain free acid **XIV.** The basic hydrolysis of the ester groups is carried out in presence of an inorganic base, preferably an alkali metal hydroxide, even more preferably in presence of sodium hydroxide.

A methanol solution was preferably employed.

Heating is preferably used; particularly refluxing methanol in presence of sodium hydroxide is used.

In step iii, free acid **XIV** is reacted with an amine of formula $X_1NH_2$ in presence of an acid-activating reagent, wherein $X_1$ is selected from H, C1-C6 alkyl, C1-C6 fluorinated alkyl and C6-C10 aryl, to obtain a compound of formula **XV.**

Preferred acid-activating reagents are HATU, oxalyl chloride or $SOCl_2$,

Preferred embodiments for the amine of formula $X_1NH_2$ were already described for the first aspect. Preferably, the amine is methylamine or a salt thereof.

In a particular embodiment, free acid **XIV** is reacted with methylamine hydrochloride in presence of HATU (Hexafluorophosphate Azabenzotriazole Tetramethyl Uronium) and DIPEA. Step iii is preferably carried out in a solvent, which may be any polar organic solvent, such as DMSO, DMF, acetone, acetonitrile, dichloromethane and N,N-dimethylacetamide. Preferably, the solvent is DMF.

Finally, in step iv, compound **XV** is subjected to acidic hydrolysis to remove the protecting group PG and obtain the compound of formula **I.** The acidic hydrolysis is preferably carried out with inorganic acids, more preferably with HCl. Step iv is preferably carried out in alcohol solvents, such as methanol. The reaction mixture is preferably heated under refluxing conditions. Thus, in a most preferred embodiment, the acidic hydrolysis of step iv is carried out with HCl in refluxing methanol. Preferably, HCl is used as an aqueous solution such as a HCl solution with an HCl concentration comprised between 1N and 5N, preferably between 3N and 5N, more preferably 4N.

The mixture may then be subjected to known work-up procedures for isolation of the product. In an embodiment, cooling down to rt is carried out. Addition of a inorganic base, such as potassium carbonate, is performed so as the pH raises to at least 8, preferably to at least 9, more preferably at about 9. The resulting mixture may be subjected to at least 1 extraction with an organic solvent, such as EtOAc; optionally, the organic phase or, if more than 1 extraction is performed, the combined organic phases are washed with brine, dried and the organic solvent is evaporated. The compound of formula I is therefore obtained as solid. Further purification, i.e. by column chromatography or (re)crystallization may be carried out to increase the product purity. In an embodiment, the purity of the compound of formula I is at least 95%, at least 96%, at least 97%, at least 98%, preferably the purity is about 99%, about 99.5%, or even about 99.9%.

The reaction time for each step of the method of the third aspect may vary depending on other variables such as the temperature, thus a skilled person would be able to predict and carefully monitor the reaction process (for example by TLC or GC-MS).

In a particular embodiment, the method of the third aspect further comprises, prior to steps i-iv as defined earlier, the steps of:

a) subjecting a compound of formula **VII** to a crossed [2+2] photocycloaddition, optionally in an enantioselective manner, in presence of a rhodium catalyst, to obtain compound **VIII:**

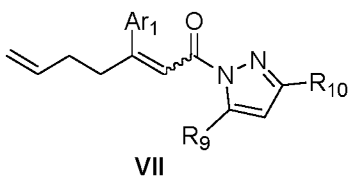

**VII**

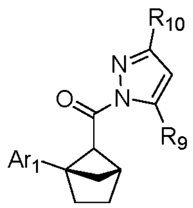

**VIII** ,

wherein $R_9$ and $R_{10}$ are independently selected from H, C1-C6 alkyl and C6-C10 aryl and $Ar_1$ is a C6-C10 (hetero)aryl group;

b) subjecting compound **VIII** to esterification with an alcohol of formula $R_{11}OH$ in presence of an inorganic chloride salt and tertiary amine, wherein $R_{11}$ is a C1-C6 alkyl or a C6-C10 aryl group, to obtain a compound of formula **IX:**

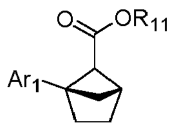

**IX** ;

c) oxidizing compound **IX** to compound **X** in the presence of a Ru(III) salt and an oxidant,

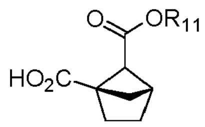

**X** ;

d) derivatization of the carboxylic group of compound X, to obtain compound **XI:**

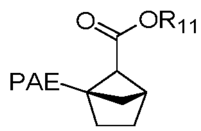

**XI**

wherein PAE is a photoredox-active ester and $R_{11}$ is defined as above.

In step a), the crossed [2+2] photocycloaddition is a photochemical reaction involving the two alkene groups (one internal and one terminal) of compound **VII** to form the cyclobutane ring in compound **VIII:**

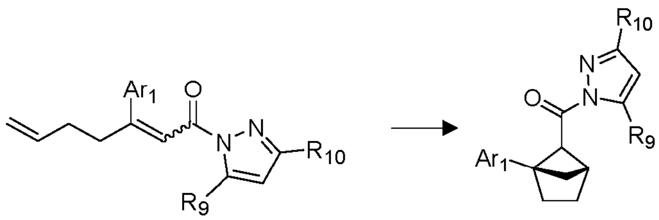

**VII** **VIII** .

wherein $R_9$ and $R_{10}$ are independently selected from H, C1-C6 alkyl and C6-C10 aryl and $Ar_1$ is a C6-C10 (hetero)aryl.

Compounds of formula **VII** have been previously described in Pablo Garrido-García et al., ChemRxiv, 2023, DOI: 10.26434/chemrxiv-2023-f5ll4. The stereochemistry of the internal double bond is not limiting in any way the efficiency of step a.

Said step is performed in the presence of a rhodium catalyst. Said rhodium catalyst may be achiral or chiral. Achiral rhodium catalysts suitable for these reactions would be octahedral rhodium catalysts of general formula:

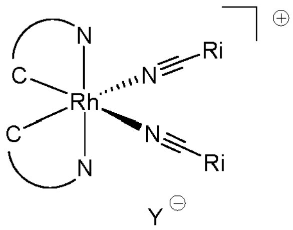

,

wherein $Y^-$ is an inorganic monovalent anion, C⌒N is a bidentate ligand coordinated to rhodium via a carbon and a nitrogen atom and Ri is a C1-C6 alkyl, preferably methyl. Such catalysts may be commercially available or may be easily obtained by reaction of a commercially available rhodium precursor with the bidentate ligand and the nitrile N≡C-Ri, for example as described by Eric Meggers and coworkers in Nat. Protoc. 13, 605-632 (2018).

Preferably, the achiral rhodium catalyst has general formula:

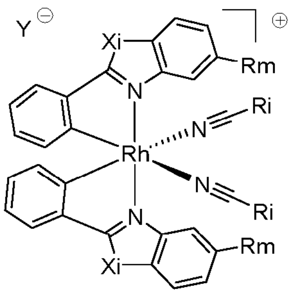

wherein:

- both Xi are independently selected from O and S, preferably they are both S;
- Ri and Rm are, independently, a C1-C6 alkyl, preferably a C1-C4 alkyl, more preferably Ri is methyl and Rm is t-butyl; and
- $Y^-$ is a monovalent anion, preferably an inorganic monovalent anion selected from $BF_4^-$, $PF_6^-$, $SbF_6^-$, more preferably $PF_6^-$.

More preferably, the achiral rhodium catalyst has the following formula:

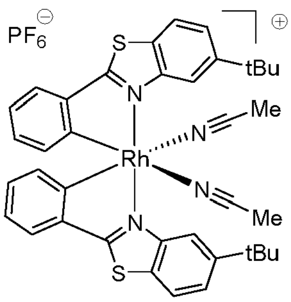

Preferably, the rhodium catalyst is chiral so as the photocycloaddition is performed in an enantioselective manner to obtain enantioenriched compound **VIII.**

In a preferred embodiment, the chiral rhodium catalyst is a chiral-at-metal rhodium catalyst, preferably with the following general formula:

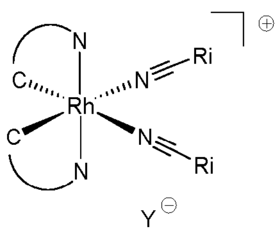

wherein $Y^-$ is an inorganic monovalent anion, 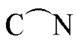 is a bidentate ligand coordinated to rhodium via a carbon and a nitrogen atom and Ri groups are independently a C1-C6 alkyl, preferably a C1-C4 alkyl, more preferably methyl. These chiral-at-metal rhodium catalysts may be obtained by known chiral resolution techniques such as chiral chromatography, that is, loading the racemic rhodium catalyst onto a column packed with a chiral stationary phase (chiral HPLC or chiral GC) and performing the elution.

Alternatively, the racemic Rh catalyst may be resolved by reaction with an enantiopure chiral auxiliary (an additional ligand which displaces the two nitrile ligands N≡C-Ri). Two diastereoisomeric complexes are obtained accordingly and these may be separated by known techniques, such as selective precipitation and centrifugation (due to different solubility), crystallization, and chromatography among other techniques. Once the diastereoisomeric complexes are separated, the chiral auxiliary may be removed and exchanged again with the nitrile ligands. A detailed procedure of chiral resolution can be found in Eric Meggers and coworkers in Nat. Protoc. 13, 605-632 (2018).

Preferably, the chiral rhodium catalyst has general formula:

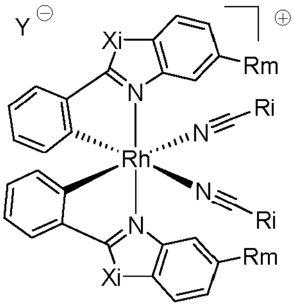

or

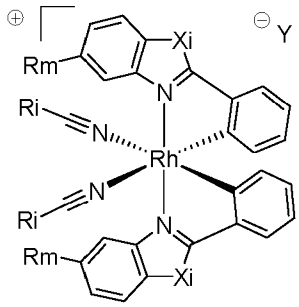

wherein both Xi, Ri and Rm and $Y^-$ are defined as above.

In a most preferred embodiment, the rhodium catalyst is:

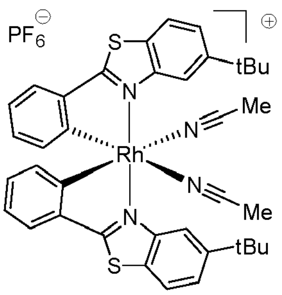

**Λ–RhS**

or

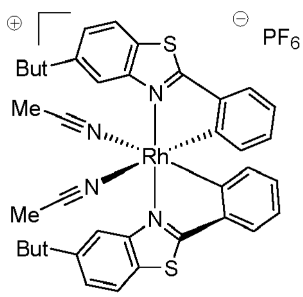

**Δ-RhS**

,

wherein the absolute configuration may be A (left complex or Λ-RhS) or Δ (right complex or Δ -RhS), according to the known CIP (Cahn-Ingold-Prelog) priority rules adapted for metal complexes with chelating ligands (Δ and A notation are based on the twist of the chelate rings, - right-handed and left-handed, respectively).

Step a) can be carried out in several organic solvents; preferably the solvent is acetone.

The photochemical reaction is performed under light irradiation, preferably in a wavelength range between 400 and 500 nm. The light source can be any known by those skilled in the art, for example light emitting diode (LED).

In a particular embodiment, compound **VII** is dissolved in acetone at room temperature under LED irradiation, preferably at a $\lambda$ = 440 nm.

In step b), compound **VIII** is subjected to esterification with an alcohol of formula $R_{11}$ OH in the presence of an inorganic chloride salt and tertiary amine, wherein $R_{11}$ is a C1-C6 alkyl or a C6-C10 aryl group, to obtain a compound of formula **IX.**

Preferably $R_{11}$ is a C1-C4 alkyl, more preferably ethyl or methyl, even more preferably methyl.

The inorganic chloride salt is preferably a chloride of an alkali metal, more preferably is LiCl. The tertiary amine is selected from trimethyl amine, triethylamine, tripropylamine, tributylamine, and ethyl diisopropyl amine; preferably, the teriary amine is triethylamine. Many organic solvents can be used for step ii, particularly ether solvents are suitable. Preferably, the solvent is a cyclic ether, such as dioxane and tetrahydrofuran; more preferably the solvent is tetrahydrofuran.

In step c), compound **IX** is oxidized to compound **X** in the presence of a Ru(III) salt, preferably a Ru(III) halide, more preferably $RuCl_3$, even more preferably hydrated $RuCl_3$, and an oxidant, preferably an oxidant selected from potassium permanganate ($KMnO_4$), potassium dichromate ($K_2Cr_2O_7$), chromium trioxide (CrOs), hydrogen peroxide ($H_2O_2$), sodium hypochlorite (NaOCl), sodium periodate ($NaIO_4$), peroxydisulfuric acid ($H_2S_2O_8$), Iodine ($I_2$), Bromine ($Br_2$), Chlorine ($Cl_2$), Oxygen ($O_2$), Ferric chloride ($FeCl_3$), lead dioxide ($PbO_2$), Osmium tetroxide ($OsO_4$), Manganese dioxide ($MnO_2$). Preferably the oxidant is $NaIO_4$.

Polar solvents are preferred for step c). Examples include water, acetonitrile, dichloromethane, chloroform, dimethylsulfoxide, tetrahydrofuran, dioxane, dimethylformamide and any mixtures thereof. Preferably, step c) is carried out in a mixture of water, acetonitrile and dichloromethane.

Thus in a preferred embodiment of step c), compound **IX** is oxidized to compound **X** in the presence of $RuCl_3$ and $NaIO_4$ in a mixture of water, acetonitrile and dichloromethane.

In step d), a derivatization of the carboxylic group of compound **X** is carried out. As used in this description, the derivatization of step d) implies the conversion of the carboxylic group of compound **X** into a photoredox-active ester (PAE)

of formula **XI** through ester-bond-forming reactions. Preferred PAEs are N-hydroxyphthalimide (NHPI), thioesters, benzyl esters and acyloxime esters. Preferably, compound **X** is reacted with phthalimide or a derivative thereof as already described in presence of an acyl chloride, such as oxalyl chloride, to obtain a compound **XI** with the following structure:

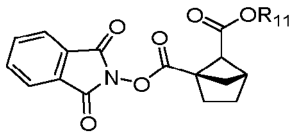

wherein $R_{11}$ is a is a C1-C6 alkyl or a C6-C10 aryl group, preferably a C1-C4 alkyl, more preferably ethyl or methyl, even more preferably methyl.

Step d) may be carried out in several organic solvents, particularly in chlorinated solvents. Preferably, the solvent in step d is dichlorometane.

Once compound **XI** is obtained in step d, steps i-iv (and all the corresponding embodiments) as described above are followed in order to obtain the compounds of formula **I.**

In an even more particular embodiment, the method according to the third aspect comprises the steps of:

i. Reacting an alkyne of formula **II** with the organomagnesium compound of formula III:

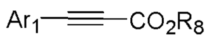
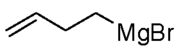

**II** **III** ,

to obtain a compound of formula IV:

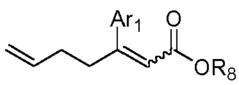

**IV** ,

wherein $Ar_1$ is a C6-C10 (hetero)aryl, preferably a C6 aryl, and Rs is a C1-C6 alkyl group or a C6-C10 aryl group, preferably a C1-C6 alkyl;

ii. Hydrolysis under basic conditions of the ester group of compound **IV** to obtain compound **V:**

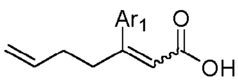

**V** ;

iii. Coupling of compound **V** with compound **VI:**

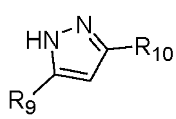

**VI** ,

to obtain compound **VII**

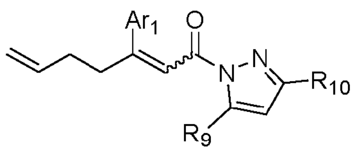

**VII**

wherein $R_9$ and $R_{10}$ are independently selected from H, C1-C6 alkyl and C6-C10 aryl, preferably wherein $R_9$ and $R_{10}$ are a C6 aryl independently on each other;

iv. Subjecting compound **VII** to a crossed [2+2] photocycloaddition, optionally in an enantioselective manner, in presence of a rhodium catalyst to obtain compound **VIII:**

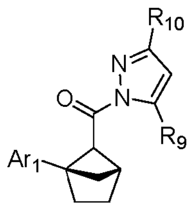

**VIII** ;

v. Subjecting compound **VIII** to esterification with an alcohol of formula $R_{11}OH$ in presence of an inorganic chloride salt and tertiary amine, wherein $R_{11}$ is a C1-C6 alkyl or a C6-C10 aryl, to obtain a compound of formula **IX:**

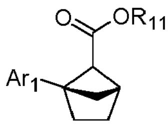

**IX** ;

vi. Oxidizing compound **IX** to compound **X** in the presence of a Ru(III) salt, preferably $RuCl_3$, and an oxidant, preferably $NaIO_4$,

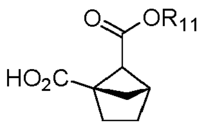

**X** ;

vii. Derivatization of compound **X,** preferably with phthalimide in presence of oxalyl chloride, to obtain compound **XI**

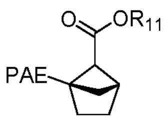

**XI**

Wherein PAE is a photoredox-active ester;

viii. Reacting compound **XI** with compound **XII** under light irradiation and in the presence of a base to obtain compound **XIII:**

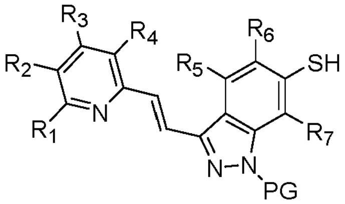

**XII** ,

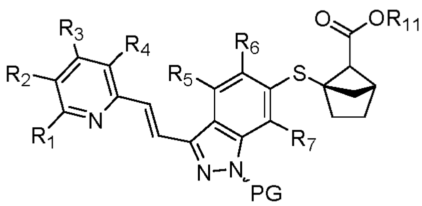

**XIII** ,

wherein $R_{11}$ is as defined above; PG is a N-protecting group; and $R_1$-$R_7$ are independently selected from H, $^2H$, halogen, C1-C6 alkyl, C1-C6 fluorinated alkyl and C6 aryl;

ix. Ester hydrolysis of compound **XIII** under basic conditions to obtain free acid **XIV:**

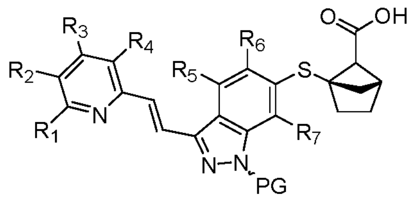

**XIV** ;

x. Reacting free acid **XIV** with an amine of formula $X_1NH_2$ in presence of an acid-activating reagent, preferably oxalyl chloride or $SOCl_2$, wherein $X_1$ is selected from H, C1-C6 alkyl, C1-C6 fluorinated alkyl and C6-C10 aryl, to obtain a compound of formula **XV:**

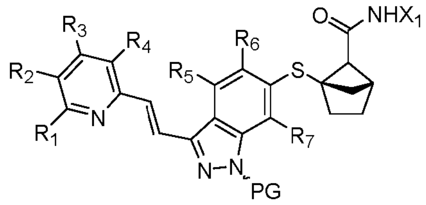

**XV** ;

xi. Subjecting compound **XV** to acidic hydrolysis to obtain compound **I.**

Particular embodiments for steps iv-xi have been already described above.

Medical uses

Another aspect of the present invention relates to a compound of formula **I,** preferably a compound of formula **Ia** and/or **Ib,** more preferably a compound of formula **Ia'** and/or **Ib',** according to the first aspect, or to a pharmaceutical composition according to the second aspect, for use in medicine.

Alternatively, the invention also refers to the use of a compound of formula **I,** preferably a compound of formula **Ia** and/or **Ib,** more preferably a compound of formula **Ia'** and/or **Ib',** according to the first aspect, or of a pharmaceutical composition according to the second aspect, in medicine.

Alternatively, the invention also refers to the use of a compound of formula **I,** preferably a compound of formula **Ia** and/or **Ib,** more preferably a compound of formula **Ia'** and/or **Ib',** according to the first aspect or to the use of a pharmaceutical composition according to the second aspect in the manufacturing of a medicament.

Particularly, the invention refers to a compound of formula **I,** preferably a compound of formula **Ia** and/or **Ib,** more preferably a compound of formula **Ia'** and/or **Ib',** according first aspect and to a pharmaceutical composition according to the second aspect for use in the treatment of cancer. In an embodiment, the cancer comprises uveal melanoma, breast cancer and colon cancer.

Alternatively, the invention also refers to the use of a compound of formula **I,** preferably a compound of formula **Ia** and/or **Ib,** more preferably a compound of formula **Ia'** and/or **Ib',** according to the first aspect, or to the use of a pharmaceutical composition according to the second aspect, in the treatment of cancer, preferably in the treatment of uveal melanoma, breast cancer and colon cancer.

Alternatively, the invention also refers to the use of a compound of formula **I,** preferably a compound of formula **Ia** and/or **Ib,** more preferably a compound of formula **Ia'** and/or **Ib',** according to the first aspect,or of a pharmaceutical composition according to the second aspect, in the manufacturing of a medicament for treating cancer, preferably for treating uveal melanoma, breast cancer and colon cancer.

Alternatively, the invention also refers to a method of treating cancer, particularly uveal melanoma, breast cancer and colon cancer, said method comprising administering to a patient in need thereof, a therapeutically effective amount of a compound of formula **I,** preferably a compound of formula **Ia** and/or **Ib,** more preferably a compound of formula **Ia'** and/or **Ib',** according to the first aspect or of a pharmaceutical composition according to the second aspect.

## EXAMPLES

General information. All reactions sensitive to air or moisture were carried out in flame-dried glassware under argon pressure using standard Schlenk techniques. Tetrahydrofuran (THF), toluene and acetonitrile (MeCN) were purified by passing through a Pure Solv™ column drying system from Innovative Technology, Inc. Dry acetone, dimethylformamide (DMF), dimethylsulfoxide (DMSO), and diethyl ether were purchased from Acros Organics and employed without further purification. Commercially available reagents and chemicals have been purchased from: Merck, TCI Chemicals, Acros Organics, Fluorochem, Activate Scientific, Apollo Scientific or BLDpharm (specified in each case).

The enantiopure **Λ-RhS** catalyst employed in the enantioselective photocatalytic reactions was obtained following the detailed experimental procedure reported by Meggers and coworkers (J. Ma et al., Nat. Protoc. 2018, 13, 605-632). The other enantiomer of the catalyst **(Δ-RhS)** can be obtained through the same procedure. Axitinib ($sp^2$-drug) was prepared according to the procedure in L.-H. Zhai et al., Org. Process Res. Dev. 2015, 19, 849-857. Compound (E)-6-iodo-3-[2-(pyridin-2-yl)ethenyl]-1-(tetrahydro-2H-pyran-2-yl)-1H-indazole was purchased from BLDpharm. Flash column chromatography was performed on silica gel 60 (VWR, 230-400 mesh) or on RediSep® Bronze columns (Teledyne, ISCO, 230-400 mesh) with the indicated eluent mixtures. Thin layer chromatography (TLC) was performed on silica-coated aluminum plates (silica 60 F254) with detection by UV-light ($\lambda$ = 254 nm) and employing potassium permanganate ($KMnO_4$) or cerium ammonium molybdate (CAM) stain developer solutions followed by heat treatment.

NMR spectra were acquired on a Bruker Avance 300 MHz spectrometer running at 300, 75, and 282 MHz for $^{1}H$, $^{13}C$, and $^{19}F$, respectively or a 500 MHz spectrometer running at 500 and 126 MHz for $^{1}H$ and $^{13}C$ respectively. Chemical shifts ($\delta$) are reported in ppm relative to the respective residual solvent signals of $CDCl_3$ [$\delta$ (1H) = 7.26 ppm, $\delta$ ($^{13}C$) = 77.16 ppm], acetone-d6 [$\delta$ ($^{1}H$) = 2.05 ppm, $\delta$ ($^{13}C$) = 29.84 ppm], DMSO-d6 [$\delta$ ($^{1}H$) = 2.50 ppm, $\delta$ ($^{13}C$) = 39.52 ppm] or $CD_2Cl_2$ [$\delta$ ($^{1}H$) = 5.32 ppm, $\delta$ ($^{13}C$) = 53.84 ppm]. Coupling constants are given in Hz. The following abbreviations are used to indicate the multiplicity: s, singlet; d, doublet; t, triplet; q, quartet; p, pentet; h, sextet; hept, heptet; m, multiplet; br, broad signal. $^{13}C$ NMR and $^{19}F$ spectra were acquired on a broad band decoupled mode. Specific rotation was determined on an Anton Paar MCP 100 polarimeter in a 10 cm path length cell and in $CHCl_3$ or MeOH (concentration in g/100 mL). Enantiomeric ratios were determined by HPLC on chiral columns on an Agilent Technologies 1260 Infinity Series instrument, employing Daicel Chiralpak IA-3, IBN-3, IC-3, and IG-3 columns and a UV-Vis detector. High Resolution Mass Spectrometry (HRMS) were registered in a GCT Agilent Technologies 6890 N spectrometer using Electronic Impact (EI+) techniques at 70 eV and electrospray (ESI+) or Bruker maXis IITM (APCI+). Melting points were determined in a StuartTM melting point SMP3 apparatus in open capillary tubes.

Gas Chromatography (GC) analysis and Low-Resolution Mass Spectrometry (LRMS) was performed on an Agilent 7820A gas chromatograph using an Agilent HP-5MS UI column with a 5977A single quadrupole mass detector.

Example 1: Synthetic routes for the synthesis of the $\alpha,\beta$-unsaturated acyl pyrazoles

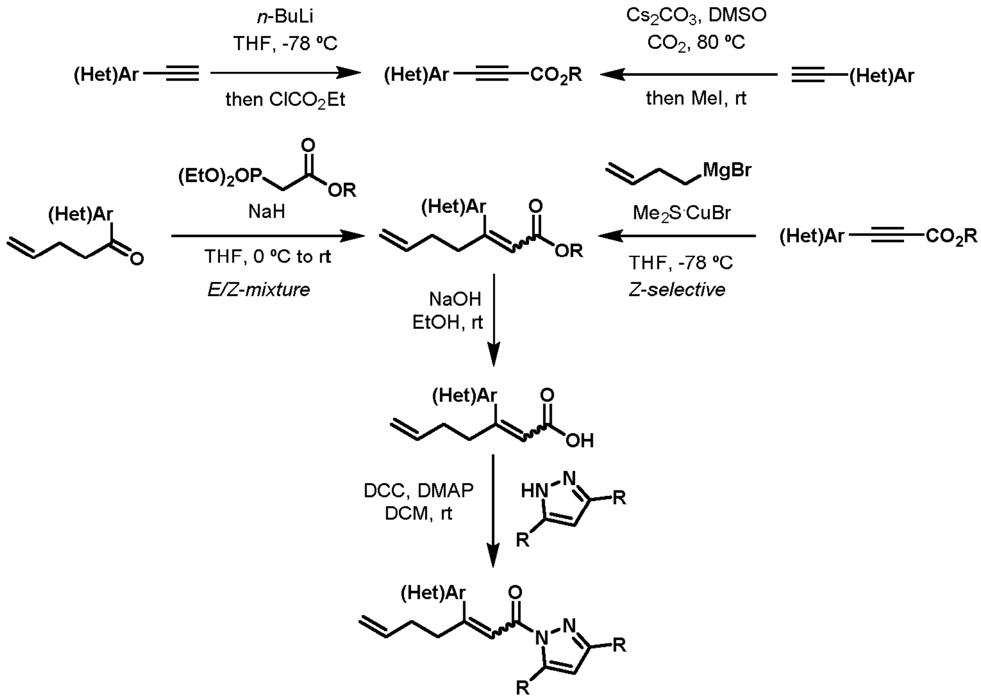


**Scheme 1.** General synthetic route towards α,β-unsaturated acyl pyrazoles

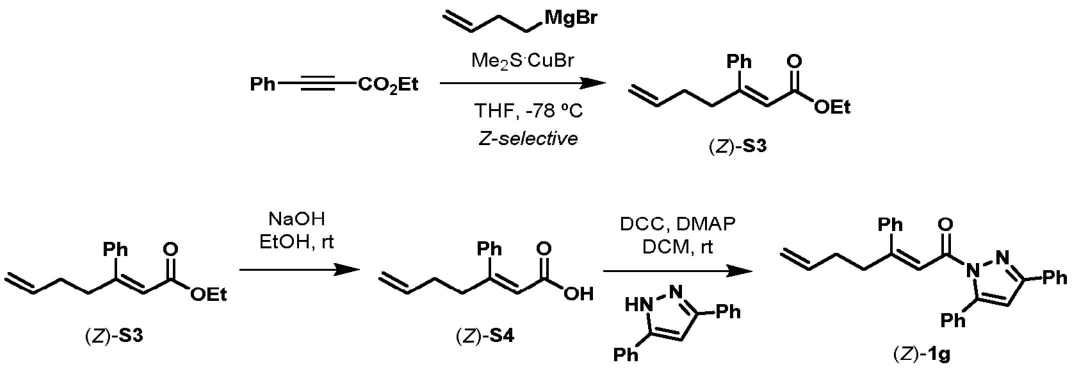


**Scheme 2.** Synthesis of compound 1g.

Experimental procedure and characterization data for compound **1g**, (*Z*)-1-(3,5-Diphenyl-1*H*-pyrazol-1-yl)-3-phenylhepta-2,6-dien-1-one (*Z*-**1g**).

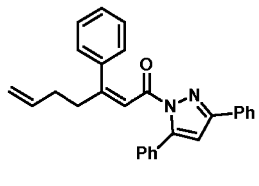

(*Z*)-**1g**

1.298 g (53.39 mmol, 3.1 equiv.) of magnesium turnings, a little crystal of iodine and 52 mL of deoxygenated THF (prepared by 15 min of argon bubbling) were placed in a two-neck round-bottom flask equipped with a reflux condenser. The resulting suspension was stirred at reflux and 6.975 g (51.66 mmol, 3 equiv.) of 4-bromobut-1-ene (supplied by *Apollo Scientific)* were gradually added to ensure a controlled formation of the corresponding Grignard reagent. After a further

hour at reflux, the Grignard solution was cooled to room temperature and dropwise added to a suspension of CuBr:dimethylsulfide complex (5.311 g, 25.83 mmol, 1.5 equiv.; supplied by *BLDpharm)* in 26 mL of THF at -78 °C. After stirring the resulting suspension at -78 °C for 20 min, a deoxygenated THF solution (17 mL) of ethyl 3-phenylpropiolate (3.000 g, 17.22 mmol; supplied by *TCI Chemicals)* was dropwise added and stirred at -78 °C for 2.5 hours. Thus, the reaction was quenched with MeOH and a saturated solution of $NH_4Cl$. The aqueous phase was extracted with diethyl ether and the combined organic phases were washed twice with brine and dried over $Na_2SO_4$. After removal of the solvents at reduced pressure the residue (*Z*)-**S3** was dissolved in 85 mL of ethanol and 34 mL of 2 N NaOH solution were added. The reaction mixture was stirred overnight at rt (room temperature) before removing the solvents at reduced pressure. The resulting solid was dissolved in water and the aqueous solution was washed twice with DCM. Then, the aqueous phase was acidified with conc. HCl and extracted with DCM three times. The combined organic phases were washed with brine and dried over $Na_2SO_4$. After removal of the solvents at reduced pressure 3.160 g of the crude acid (*Z*)-**S4** were obtained as a white solid (91% yield, 2 steps). The latter (3.160 g, 15.62 mmol) was dissolved in 40 mL of DCM and 3.868 g (18.75 mmol, 1.2 equiv.; Apollo Scientific) of DCC and 190.9 mg (1.56 mmol, 0.1 equiv.; supplied by *TCI Chemicals)* of DMAP were added, followed by the addition of 4.130 g (18.75 mmol, 1.2 equiv.; supplied by BLDpharm) of 3,5-diphenylpyrazole. The reaction mixture was stirred at rt overnight before being filtered and rinsed with DCM. After removal of the solvents at reduced pressure the residue was purified by flash chromatography on silica gel (two different columns: from 0 to 3% EtOAc in cyclohexane and from 0 to 5% $Et_2O$ in *n*-pentane) to obtain 3.410 g of product (*Z*)-**1g** as a white solid (54% yield). The stereochemical assignment of (*Z*)-**1g** was determined upon analysis of the corresponding NOESY spectra of (*Z*)-**S3**.

$\mathbf{R_f}$= 0.30 (4% EtOAc in cyclohexane).

mp = 85 °C

**$^1$H NMR** (300 MHz, $CDCl_3$) $\delta$ 8.10 - 8.00 (m, 2H), 7.63 - 7.29 (m, 14H), 6.79 (s, 1H), 6.00 (ddt, *J*= 16.8, 10.2, 6.5 Hz, 1H), 5.28 - 5.09 (m, 2H), 2.86 - 2.75 (m, 2H), 2.43 - 2.30 (m, 2H).

**$^{13}$C NMR** (75 MHz, $CDCl_3$) $\delta$ 163.8, 160.4, 152.9, 147.4, 139.8, 137.3, 132.1, 131.1, 129.1, 128.9 (4C), 128.6, 128.2 (2C), 128.01, 127.98 (2C), 127.4 (2C), 126.4 (2C), 118.6, 115.6, 109.6, 40.2, 31.7.

**HRMS (ESI)** = calculated for $C_{28}H_{24}N_2NaO^+$, $[M+Na]^+$ = 427.1781; found = 427.1779.

Example 2: Setup and general procedure for the enantioselective crossed [2+2] photocycloaddition

The photochemical reactions were carried out through irradiation of the reaction vials from the side (see Figure 1) employing a commercially available light source (Kessil PR160L, $\lambda_{max}$ = 440 nm) from a 5 cm distance. The temperature of the reaction mixture was kept at room temperature employing a set of fans.

An oven-dried Schlenk tube, equipped with a magnetic stir bar, was charged with catalyst **A-RhS** (1.7 mg, 2.00 $\mu$mol), (*Z*)-$\alpha$,$\beta$-unsaturated acyl pyrazole **1** (0.100 mmol) and 1.0 mL of dry acetone (0.1 M). The reaction mixture was stirred at room temperature under blue LED irradiation ($\lambda$ = 440 nm), in the reaction setup described above and shown in Figure 1, until completion of the reaction as judged by TLC analysis (consumption of the more polar Z-isomer of **1**). The solvent was removed at reduced pressure and the crude mixture was purified by flash column chromatography on silica gel to afford the corresponding bicyclo[2.1.1]hexanes **2** in stated yield and enantiomeric purity. The same process was also scaled up using 1 mmol of compound **1** and similar results were obtained.

Synthesis of compound **2g**, (3,5-Diphenyl-1*H*-pyrazol-1-yl)((1*R*,4*R*,5*R*)-1-phenylbicyclo[2.1.1]hexan-5-yl)methanone.

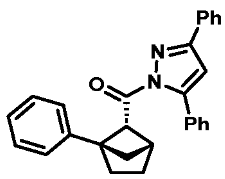

**2g**

**2g** was prepared according to the general procedure (starting from compound **(Z)-1g)** after 12 hours of irradiation and purified by flash chromatography on silica gel (from 0 to 5% EtOAc in cyclohexane) to obtain 39.0 mg of product as a white solid (96% yield). The enantiomeric ratio (98:2 *er)* was determined by normal phase HPLC on a *Daicel Chiralpak* IBN-3 column [$t_{major}$ = 5.3 min, $t_{minor}$ = 4.5 min; flow rate 1.0 mL/min; i-PrOH:n-hexane = 1:99; $\lambda$ = 250 nm].

$\mathbf{R_f}$ = 0.28 (4% EtOAc in cyclohexane).

$[\alpha]^{20}_D$ = - 104 (c = 1.00, $CHCl_3$).

**mp** = 96 - 98 °C

**$^1$H NMR** (300 MHz, $CDCl_3$) δ 7.89 - 7.84 (m, 2H), 7.51 - 7.40 (m, 10H), 7.34 - 7.26 (m, 2H), 7.24 - 7.16 (m, 1H), 6.70 (s, 1H), 3.80 (d, *J* = 3.1 Hz, 1H), 3.32 - 3.23 (m, 1H), 2.63 - 2.48 (m, 1H), 2.05 - 1.97 (m, 1H), 1.97 - 1.84 (m, 3H), 1.49 (d, *J*= 6.3 Hz, 1H).

**$^{13}$C NMR** (75 MHz, $CDCl_3$) δ 170.4, 153.2, 147.2, 142.2, 132.1, 131.3, 129.2, 129.0 (2C), 128.9 (2C), 128.8, 128.2 (2C), 128.1 (2C), 127.1 (2C), 126.5, 126.4 (2C), 109.5, 57.3, 54.3, 43.7, 42.8, 30.0, 26.9.

**HRMS (ESI)** = calculated for $C_{28}H_{24}N_2NaO^+$, $[M+Na]^+$ = 427.1781; found = 427.1779.

Absolute configuration of compound **2g** (*1R, 4R, 5R*) was determined by comparison with the absolute configuration of compound **3,** the absolute configuration of which was determined by X-ray crystallographic analysis, as reported in P. Garrido-Garcia et al., ChemRxiv 2023, DOI: 10.26434/chemrxiv-2023-f5lI4. Briefly, compound **3,** a 3,5-dinitrobenzyl ester derivative of compound **2m,** was obtained through hydrolysis of the pyrazole moiety of compound **2m,** followed by esterification (see scheme and experimental procedure below).

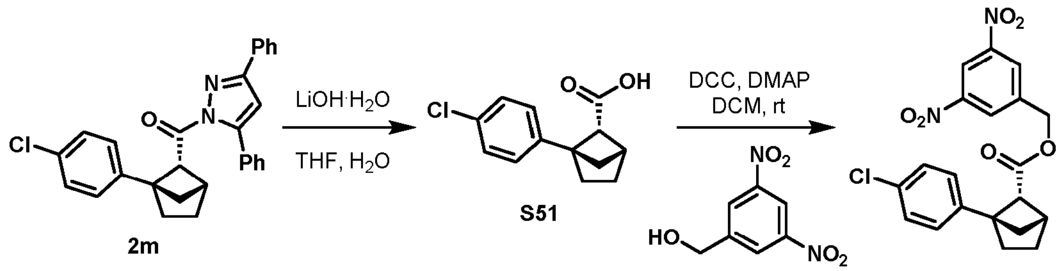


2m S51 3

Synthesis of compound **3,** 3,5-Dinitrobenzyl (1*R*,4*R*,5*R*)-1-(4-chlorophenyl)bicyclo[2.1.1]hexane-5-carboxylate

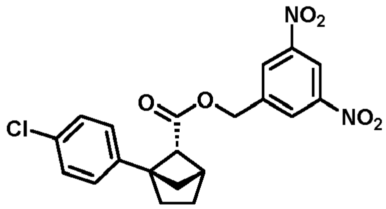

Compound **2m** was obtained starting by appropriate precursors according to the general procedures in examples 1 and 2.

87.8 mg (0.20 mmol) of **2m** were dissolved in 1.4 mL of THF and 0.7 mL of $H_2O$ before 16.8 mg (0.40 mmol, 2 equiv.) of LiOH monohydrate (Merck) were added. The reaction mixture was stirred at rt for 16 h. Thus, 10 mL of 1 N NaOH were added, and the aqueous solution was washed twice with DCM. The aqueous phase was acidified with conc. HCl and extracted with DCM three times. The combined organic phases were washed with brine and dried over $MgSO_4$. After removal of the solvents at reduced pressure 47.3 mg of the crude acid **S51** were obtained (quant, yield).

16.8 mg (0.071 mmol) **of S51** were dissolved in 1.4 mL of DCM, then 17.6 mg (0.085 mmol, 12 equiv., Apollo Scientific) of DCC and 0.9 mg (0.007 mmol, 0.1 equiv., TCI Chemicals) of DMAP were added, followed by the addition of 16.9 mg (0.085 mmol, 1.2 equiv., Merck) of (3,5-dinitrophenyl)methanol. The reaction mixture was stirred at rt overnight before being filtered and rinsed with DCM. After removal of the solvents at reduced pressure the residue was purified by flash chromatography on silica gel (from 0 to 10% EtOAc in cyclohexane) to obtain 25.0 mg of product **3** as a pale-yellow solid (85% yield).

CCDC 2286648 contains the supplementary crystallographic data of compound **3.** These data can be obtained free of charge at www.ccdc.cam.ac.uk, website of Cambridge Crystallographic Data Centre. Thus, the absolute configuration was determined to be *1R, 4R, 5R.*

**$R_f$ =** 0.14 (10% EtOAc in cyclohexane).

**$[\alpha]^{24}_D$** = -84.4 (c = 0.498, $CHCl_3$).

**mp =** 112 °C

**$^1$H NMR** (300 MHz, $CDCl_3$) δ 8.98 (t, *J*= 2.1 Hz, 1H), 8.48 - 8.42 (m, 2H), 7.34 - 7.22 (m, 4H), 5.27 - 5.23 (m, 2H), 2.90 - 2.84 (m, 1H), 2.79 - 2.73 (m, 1H), 2.17 - 2.05 (m, 1H), 1.96 - 1.82 (m, 3H), 1.80 - 1.74 (m, 1H), 1.39 (d, J= 6.7 Hz, 1H).

**$^{13}$C NMR** (75 MHz, $CDCl_3$) δ 170.2, 148.8 (2C), 140.8, 139.9, 132.7, 128.5 (2C), 128.2 (2C), 127.7 (2C), 118.6, 63.4, 57.1, 52.8, 42.3, 40.1, 29.6, 26.5.

**HRMS (ESI)** = calculated for $C_{20}H_{16}ClN_2O_6^-$, $[M-H]^-$ = 415.0702; found = 415.0709.

Example 3: Synthesis and characterization data of compound 12

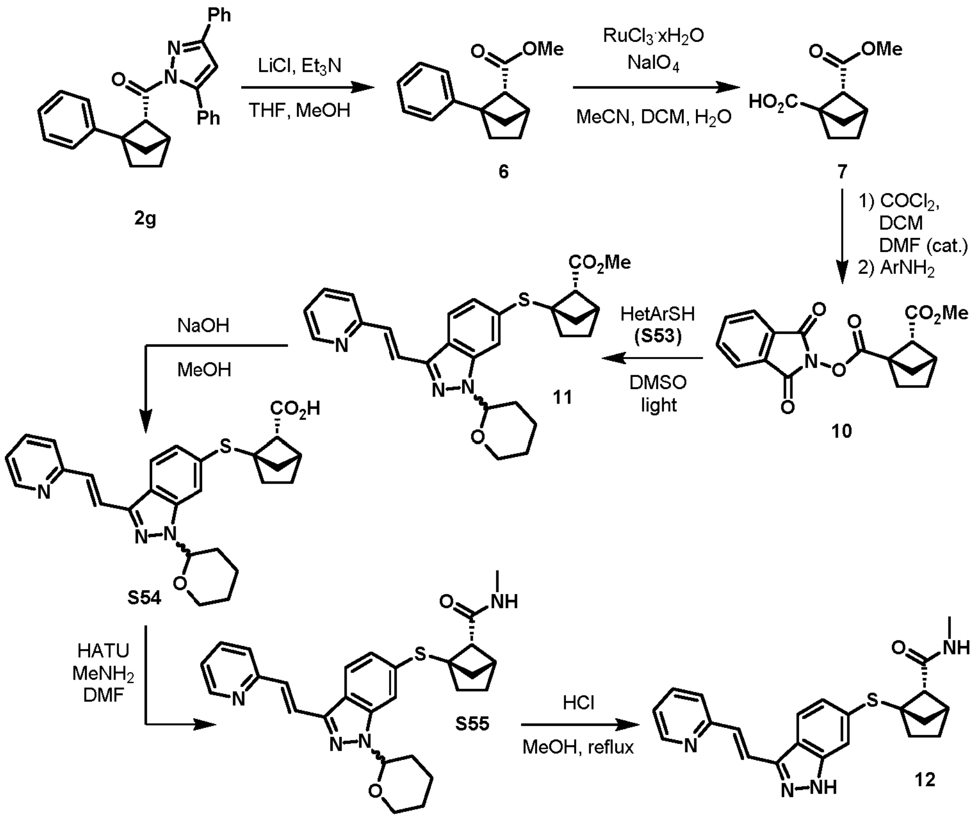


**Scheme 3.** Synthesis of compound **12**.

**Synthesis of Methyl (1R,4R,SR)-1-phenylbicyclo[2.1.1]hexane-5-carboxylate (6)**

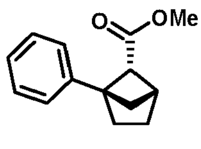

**6**

1.048 g (2.59 mmol) of **2g** were dissolved in 20 mL of MeOH and 5 mL of THF. Thus, 549.2 mg (12.96 mmol, 5 equiv.; supplied by *TCI Chemicals)* of anhydrous LiCl were added, followed by 1.8 mL (12.96 mmol, 5 equiv.; supplied by *Fluorochem)* of $Et_3N$ and the resulting reaction mixture was stirred at rt overnight (14 h). Solvents were removed under reduced pressure, and the residue was purified by flash chromatography on silica gel (from 0 to 3% EtOAc in cyclohexane) to obtain 544.3 mg of product 6 as a colorless oil (97% yield).

$\mathbf{R_f}$ **=** 0.28 (4% EtOAc in cyclohexane).

$[\alpha]^{24}_D$ = - 129 (c = 0.500, $CHCl_3$).

$^1$**H NMR** (300 MHz, $CDCl_3$) δ 7.45 - 7.37 (m, 2H), 7.37 - 7.29 (m, 2H), 7.28 - 7.19 (m, 1H), 3.66 (s, 3H), 2.87 - 2.76 (m, 1H), 2.74 - 2.62 (m, 1H), 2.32 - 2.16 (m, 1H), 2.03 - 1.91 (m, 1H), 1.91 - 1.77 (m, 2H), 1.77 - 1.68 (m, 1H), 1.38 (d, *J*= 6.5 Hz, 1H).

$^{13}$**C NMR** (75 MHz, $CDCl_3$) δ 171.7, 142.1, 128.3 (2C), 126.7 (2C), 126.6, 57.3, 52.9, 51.2, 42.0, 39.9, 30.0, 26.7.

**HRMS (ESI)** = calculated for $C_{14}H_{17}O_2^+$, $[M+H]^+$ = 217.1223; found = 217.1224.

**Synthesis of (1R,4R,SR)-5-(Methoxycarbonyl)bicyclo[2.1.1]hexane-1-carboxylic acid (7)**

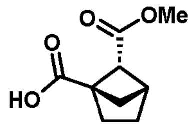

7

560.3 mg (2.59 mmol) of 6 were dissolved in a 2:2:3 mixture of MeCN:DCM:$H_2O$ (52 mL), to which 29.2 mg (0.130 mmol, 0.05 equiv.; supplied *by Merck)* of $RuCl_3$ $xH_2O$ were added, followed by sodium periodate (11.08 g, 51.81 mmol, 20 equiv.; Merck). After vigorously stirring the suspension for 14 hours a 1 N NaOH solution was added to ensure basic pH and the mixture was filtered, rinsing with water. The filtrate was transferred to a separation funnel and the aqueous phase was washed twice with diethyl ether. The organic phase was extracted with 1 N NaOH solution and the combined aqueous phases were acidified with conc. HCl. Thus, the acidified aqueous layer was extracted four times with DCM, and the combined organic phases were washed with brine and dried over $Na_2SO_4$. After removal of the solvents at reduced pressure 379.5 mg of crude product 7 (80% yield) were obtained as a black solid, which was used in the next step without further purification.

**$^1$H NMR** (300 MHz, $CDCl_3$) δ 9.35 (br s, 1H), 3.71 (s, 3H), 2.82 (d, *J* = 3.1 Hz, 1H), 2.75 (tt, *J* = 2.8, 1.4 Hz, 1H), 2.08 - 1.96 (m, 1H), 1.96 - 1.87 (m, 1H), 1.84 - 1.71 (m, 3H), 1.35 (d, *J* = 6.8 Hz, 1H).

**Synthesis of 1-(1,3-Dioxoisoindolin-2-yl) 5-methyl (1*R*,4*R*,5*R*)-bicyclo[2.1.1]hexane-1,5-dicarboxylate (10)**

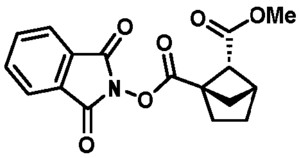

10

To a solution **of 7** (353.0 mg, 1.916 mmol) in dry DCM (10 mL) were added 252 μL (2.875 mmol, 1.5 equiv.; supplied by *TCI Chemicals)* of oxalyl chloride and two drops of dry DMF. The mixture was stirred at rt for 2 h and concentrated at reduced pressure. The residue was taken up in dry DCM (10 mL), and cooled to 0 °C. Thus, N-hydroxyphthalimide (375.2 mg, 2.300 mmol, 1.2 equiv.; supplied by Merck) in dry DMF:DCM (1:9 - 10 mL) was added, followed by the dropwise addition of 534 μL (3.833 mmol, 2 equiv.; supplied by *Fluorochem)* of $Et_3N$. The reaction mixture was let warm to rt and stirred overnight before being quenched with water and extracted with DCM two times. The combined organic layers were washed with brine twice, and dried over $Na_2SO_4$. After removal of the solvents at reduced pressure the residue was purified by flash chromatography (from 0 to 50% EtOAc in cyclohexane) to obtain 485.3 mg of redox-active ester **10** as a yellowish sticky solid (77% yield).

**$R_f$ =** 0.36 (33% EtOAc in cyclohexane).

$[\alpha]^{20}_D$ = - 87.5 (*c* = 1.00, $CHCl_3$).

**$^1$H NMR** (300 MHz, $CDCl_3$) δ 7.90 - 7.83 (m, 2H), 7.81 - 7.73 (m, 2H), 3.70 (s, 3H), 3.05 - 2.99 (m, 1H), 2.84 (tt, *J* = 3.0, 1.4 Hz, 1H), 2.23 (dddd, *J* = 11.3, 8.7, 4.2, 2.5 Hz, 1H), 2.14 - 1.97 (m, 2H), 1.96 - 1.76 (m, 2H), 1.49 (d, *J* = 6.6 Hz, 1H).

**$^{13}$C NMR** (75 MHz, $CDCl_3$) δ 169.8, 167.6, 161.9 (2C), 134.8 (2C), 129.2 (2C), 124.0 (2C), 51.9, 51.8, 51.3, 41.3, 40.6, 26.4, 25.2.

**HRMS (ESI)** = calculated for $C_{17}H_{16}NO_6^+$, $[M+H]^+$ = 330.0972; found = 330.0965.

**Synthesis of (*E*)-*S*-(3-(2-(Pyridin-2-yl)vinyl)-1-(tetrahydro-2*H*-pyran-2-yl)-1*H*-indazol-6-yl)ethanethioate (S52)**

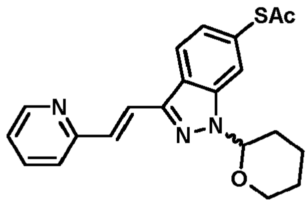

S52

A suspension of (*E*)-6-iodo-3-[2-(pyridin-2-yl)ethenyl]-1-(tetrahydro-2*H*-pyran-2-yl)-1*H*-indazole (2.724 g, 6.32 mmol; supplied by *BLDpharm),* potassium thioacetate (2.164 g, 18.95 mmol, 3 equiv. supplied by *Fluorochem),* CuI (240.6 mg, 1.26 mmol, 0.2 equiv.; supplied by *Merck),* and 1,10-phenanthroline (455.3 mg, 2.53 mmol, 0.4 equiv.; supplied *by Merck)* in dry toluene (25 mL) was heated at reflux under argon. After 44 hours full conversion was observed by TLC analysis and the reaction mixture was cooled to rt and quenched with water. The aqueous phase was extracted twice with diethyl ether and the combined organic phases were washed twice with brine and dried over sodium sulphate. After removal of the solvents at reduced pressure, the residue was purified by flash chromatography on silica gel (from 0 to 40% EtOAc in cyclohexane) to obtain 1.557 g of product **S52** (65% yield).

$\mathbf{R_f}$ **=** 0.42 (33% EtOAc in cyclohexane).

**$^1$H NMR** (300 MHz, $CDCl_3$) δ 8.63 (ddd, *J*= 4.8, 1.8, 0.8 Hz, 1H), 8.06 (dd, *J* = 8.4, 0.8 Hz, 1H), 7.89 (d, *J* = 16.4 Hz, 1H), 7.73 (dd, *J* = 1.4, 0.8 Hz, 1H), 7.68 (td, *J* = 7.7, 1.8 Hz, 1H), 7.59 (d, *J* = 16.4 Hz, 1H), 7.48 (dt, *J* = 7.9, 1.0 Hz, 1H), 7.25 (dd, *J* = 8.4, 1.4 Hz, 1H), 7.17 (ddd, *J* = 7.5, 4.8, 1.1 Hz, 1H), 5.74 (dd, *J* = 9.2, 2.7 Hz, 1H), 4.10 - 3.99 (m, 1H), 3.82 - 3.68 (m, 1H), 2.62 - 2.56 (m, 1H), 2.47 (s, 3H), 2.28 - 2.01 (m, 2H), 1.88 - 1.63 (m, 3H).

Synthesis **of (*E*)-3-(2-(Pyridin-2-yl)vinyl)-1-(tetrahydro-2*H*-pyran-2-yl)-1*H*-indazole-6-thiol (S53)**

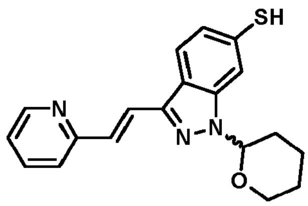

S53

To a solution **of S52** 674.0 mg (1.78 mmol) in 18 mL of MeOH was added $K_2CO_3$ (1.23 g, 8.88 mmol, 5 equiv.). After stirring for 15 min full conversion was observed by TLC analysis and the reaction mixture was acidified until pH 3 by careful addition of 1 N HCl. After stirring for 2 hours at rt, the mixture was extracted four times with DCM and the combined organic layers were washed with brine and dried over $Na_2SO_4$. After removal of the solvents at reduced pressure, crude **S53** was obtained and used in the next step without further purification.

**$^1$H NMR** (300 MHz, $CD_2Cl_2$) δ 8.59 (d, *J*= 4.8 Hz, 1H), 8.10 - 7.85 (m, 2H), 7.82 - 7.66 (m, 1H), 7.59 - 7.44 (m, 3H), 7.25 - 7.09 (m, 2H), 5.69 - 5.56 (m, 1H), 4.06 - 3.94 (m, 1H), 3.83 - 3.56 (m, 1H), 2.60 - 2.36 (m, 1H), 2.19 - 1.94 (m, 2H), 1.84 - 1.51 (m, 3H).

**Methyl (1*R*,4*R*,5*S*)-1-((3-((*E*)-2-(pyridin-2-yl)vinyl)-1-(tetrahydro-2*H*-pyran-2-yl)-1*H*-indazol-6-yl)thio)bicyclo [2.1.1]hexane-5-carboxylate (11)**

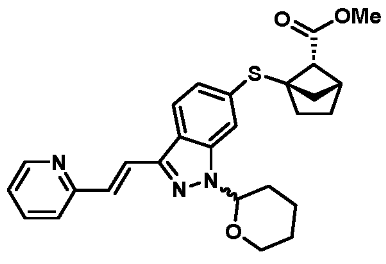

11

465.3 mg (1.413 mmol, 1 equiv.) **of 10** and 596.0 mg (1.77 mmol, 1.25 equiv.) of **S53** were dissolved in 14.1 mL of dry DMSO. Thus, 148 μL (0.848 mmol, 0.6 equiv.; supplied by *TCI Chemicals)* of DIPEA were added and the reaction mixture was deoxygenated by argon bubbling (15 min) before being stirred for 12 h under blue LED irradiation (λ = 440 nm), as illustrated in the reaction setup (Figure 1). The reaction was diluted with water and extracted three times with EtOAc. The combined organic layers were washed five times with brine and dried over $Na_2SO_4$. After removal of the solvents at reduced pressure the residue was purified by flash chromatography on silica gel (from 0 to 40% EtOAc in cyclohexane) to obtain 113.7 mg of product 11 as a mixture of diastereoisomers (17% yield).

**$R_f$ =** 0.39 (33% EtOAc in cyclohexane).

**$^1$H NMR** (300 MHz, $CDCl_3$) δ 8.62 (d, *J* = 4.8 Hz, 1H), 7.98 (d, *J* = 8.4 Hz, 1H), 7.94 - 7.81 (m, 2H), 7.68 (t, *J* = 7.7 Hz, 1H), 7.57 (d, *J* = 16.4 Hz, 1H), 7.48 (d, *J* = 7.9 Hz, 1H), 7.41 (d, *J* = 8.4 Hz, 1H), 7.20 - 7.12 (m, 1H), 5.80 - 5.63 (m, 1H), 4.05 (d, *J* = 11.6 Hz, 1H), 3.80 - 3.70 (m, 1H), 3.69 (s, 3H), 2.67 (s, 1H), 2.63 - 2.49 (m, 1H), 2.34 (s, 1H), 2.25 - 1.87 (m, 4H), 1.85 - 1.60 (m, 6H), 1.17 (dd, *J*= 6.7, 2.4 Hz, 1H).

**$^{13}$C NMR** (75 MHz, $CDCl_3$) δ 170.4, 155.7, 149.8, 142.5, 141.1, 136.7, 131.4, 130.7, 129.4, 123.7, 123.10, 123.08, 122.4, 121.9, 120.9, 118.1, 118.0, 85.9, 67.74, 67.70, 59.8, 59.7, 52.44, 52.36, 51.3, 43.23, 43.21, 38.5, 38.4, 29.7, 29.6, 29.5, 26.4, 25.2, 22.7.

**HRMS (ESI)** = calculated for $C_{27}H_{30}N_3O_3S^+$, $[M+H]^+$ = 476.2002; found = 476.2009.

**(1*R*,4*R*,5*S*)-*N*-Methyl-1-((3-((*E*)-2-(pyridin-2-yl)vinyl)-1*H*-indazol-6-yl)thio)bicyclo[2.1.1]hexane-5-carboxamide (12)**

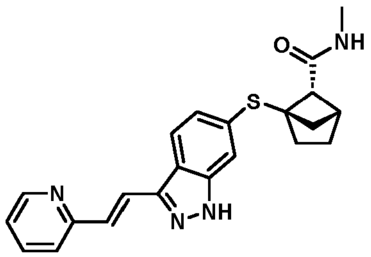

12

113.7 mg (0.239 mmol, 1 equiv.) of 11 were dissolved in 2.4 mL of MeOH and 286.9 mg (7.172 mmol, 30 equiv.) of NaOH were added. The reaction was stirred for 12 h at reflux before being cooled to rt. The pH was adjusted to 4 by addition of 1 N HCl and the aqueous phase was extracted five times with EtOAc. The combined organic phases were washed with brine and dried over $Na_2SO_4$. After removal of the solvents at reduced pressure, the crude acid **S54** (see scheme 3) was obtained as a yellow solid. The latter was dissolved in 3 mL of DMF and 109.1 mg (0.287 mmol, 1.2 equiv.; supplied by *BLDpharm)* of HATU and 32.3 mg (0.478 mmol, 2.0 equiv.; supplied *by Merck)* of methylamine hydrochloride were added, followed by the dropwise addition of 187 μL (1.076 mmol, 4.5 equiv.; supplied by *TCI Chemicals)* of DIPEA. The reaction mixture was stirred at rt for 12 h before being diluted with water and extracted five times with EtOAc. The combined organic layers were washed three times with brine and dried over $Na_2SO_4$, affording the crude amide **S55** (see scheme 3) after solvent removal at reduced pressure. To a stirred solution of crude amide in MeOH (6 mL) was added 4 N HCl (3.6 mL) and the reaction mixture was stirred at reflux for 3 hours, cooled to rt and basified by careful addition of a saturated solution of $K_2CO_3$ until pH 9. The aqueous phase was extracted five times with EtOAc and the combined organic phases were washed with brine and dried over $Na_2SO_4$. After removal of the solvents at reduced pressure the residue was purified by flash chromatography on silica gel (from 0 to 10% MeOH in DCM) to obtain 62.2 mg of product **12** as a white solid (67% yield, over three steps). The enantiomeric purity of the drug analogue (98:2 *er)* was determined by normal phase HPLC on a

*Daicel Chiralpak* IBN-3 column [$t_{major}$ = 8.3 min, $t_{minor}$ = 13.2 min; flow rate 1.0 mL/min; *i*-PrOH:*n*-hexane = 30:70; λ = 250 nm].

$R_f$ **=** 0.20 (4% MeOH in DCM).

$[\alpha]^{24}_D$ = - 153 (c = 1.00, $CHCl_3$).

**mp** = 94 °C

**$^1$H NMR** (300 MHz, $CDCl_3$) δ 12.24 (br s, 1H), 8.66 (ddd, *J* = 4.9, 1.9, 0.9 Hz, 1H), 8.00 - 7.91 (m, 2H), 7.74 - 7.65 (m, 2H), 7.60 (d, *J* = 16.4 Hz, 1H), 7.48 (dt, *J* = 7.9, 1.1 Hz, 1H), 7.26 (dd, *J* = 8.4, 1.3 Hz, 1H), 7.19 (ddd, *J* = 7.6, 4.8, 1.1 Hz, 1H), 6.30 (q, *J* = 4.8 Hz, 1H), 2.82 (d, *J* = 4.8 Hz, 3H), 2.66 (t, *J* = 3.1 Hz, 1H), 2.15 - 2.09 (m, 1H), 1.96 - 1.85 (m, 1H), 1.81 - 1.59 (m, 3H), 1.27 - 1.22 (m, 1H), 1.10 (d, *J* = 6.6 Hz, 1H).

**$^{13}$C NMR** (75 MHz, $CDCl_3$) δ 171.5, 155.6, 149.7, 143.2, 142.0, 136.9, 130.4, 130.3, 128.5, 123.9, 122.6, 122.2, 121.8, 121.2, 117.7, 58.8, 52.9, 42.2, 37.8, 29.9, 26.4, 25.9.

**HRMS (ESI)** = calculated for $C_{22}H_{23}N_4OS^+$, $[M+H]^+$ = 391.1587; found = 391.1592.

The other enantiomers of **2g** and **12** can be obtained through the same synthetic route employing the opposite enantiomer of the chiral-at-rhodium catalyst **(Δ-RhS).**

Example 4: Biological studies.

Roswell Park Memorial Institute medium (RPMI), Dulbecco's Modified Eagle's Medium (DMEM), McCoy 5A medium, streptomycin-penicillin (100X), fetal bovine serum (FBS), L-glutamine (100X), trypsin (10X), phosphate-buffered saline (PBS 1x) and cell culture plastic-ware were purchased from VWR. Propidium iodide (PI), resazurin sodium salt, and ethanol for cell culture were obtained from Sigma-Aldrich; RNase H, from Thermo Fisher Scientific.

The used cell lines were uveal melanoma cells Mel202 (provided by Dr. Susana Ortiz Urda, UCSF) cultured with RPMI; triple negative breast cancer cells MB231 (ATCC® HTB-26™), and colon cancer cells HTC116 (provided by Dr. Ana Pizarro, IMDEA Nanociencia) cultured with McCoy 5A. All the cell culture mediums were supplemented with 10% FBS, 1% streptomycin-penicillin and 1% L-glutamine to obtain complete medium. All the cell cultures were incubated at 37 °C in a Binder CB210 incubator (5% CO2). All the procedures were performed inside a laminar flow hood Bio II Advance Plus (Telstar).

**Cell viability assays.** After incubation of each compound for 24 h, cell viability studies were performed 24, 48 and 72 h after treatment. A stock solution of resazurin sodium salt (1 mg× mL-1) in PBS 1x was diluted to 1 % (v/v) in complete medium. After 3 hours of incubation in the incubator, the fluorescence was measured at 25 °C in a plate reader Synergy H4 Hybrid reader (BioTEK), λex = 550 nm, λem = 590 nm, gain 50. The fluorescent intensity measurements were processed using the following equation:

$$\%\ cell\ viability = ((sample\ data\text{-}negative\ control)/(positive\ control\text{-}negative\ control))\times 100$$

R Project for Statistical Computing (R-3.2.5) software (R Development Core Team) was employed for statistical analysis. Six replicates per condition were performed. Assuming a normal distribution, one-way ANOVA test was utilized to compare the statistical differences between the mean value of each condition and control. Significant differences were indicated in relation with p-value.* p-value < 0.05, ** p-value < 0.01, and *** p-value < 0.001.

**Cell cycle assay.** Cells were seeded and treated for 24 h. 72 h after treatment the samples were trypsinized, collected in a 15 ml tube and washed with PBS 1x by centrifugation at 177 rcf (relative centrifugal force) for 5 min in an Eppendorf centrifuge 5804 R. Then, the cells were fixed for 10 min with 70% cold methanol and centrifuged for 15 min. After that, cells were washed with PBS 1x and 5 min 177 rcf centrifugation. Finally, the supernatants were discarded and each sample was treated with 10 μl RNAsa A 20 mg/ml (Thermo Fisher Scientific) and 5 μl propidium iodide (PI) (Sigma Aldrich) (1 mg/ml) in a final volume of 500 μl in PBS. Cell cycle analysis was performed in a Beckman Coulter Cytomics 500 Flow Cytometer using 20000 cells. These experiments were performed in the Flow Cytometry Service at the CNB-CSIC. The acquired data was analysed with FCS Express 7 software, the cell cycle was adjusted with the multicycle model 5.

**Cell death assay.** Cells were seeded and treated for 24h. 72h after the treatment the supernatants were collected and the samples were trypsinized and mixed with the supernantants. The samples were washed with PBS 1x and centrifuged at 177 rcf for 5 min in an Eppendorf centrifuge 5804 R. After that the cells were resuspended in 100 μl 1x Annexin Binding Buffer (Thermo Fisher Scientific) and 5 μl FITC Annexin V (BioLegend) was added. It was incubated for 10 min at 4°C, avoiding light exposure. Then, 400 μl 1x Annexin Binding Buffer was added. Finally, 5 μl 1mg/ml PI was added. Cell death analysis was performed in a Beckman Coulter Cytomics 500 Flow Cytometer using 20000 cells. Controls with only annexin V or only PI were employed to adjust the gates. The acquired data was analysed with FCS Express 7 software.

**Results.** The absolute configuration determines the overall shape and molecular architecture of a specific drug analogue, enabling a different interaction of the two enantiomers in biological complex systems. As a consequence, the biological activity of the two enantiomers of drug analogue **12,** obtained in a stereocontrolled manner, was tested in line

with the regulations on chiral drugs of the FDA and EMA (W. H. Brooks et al., Curr. Top. Med. Chem., 2011, 11, 760-770). As can be observed in Figure 4, the enantiomeric saturated analogues surprisingly exhibited higher toxicity than the $sp^2$-drug Axitinib, showcasing that the replacement of a flat scaffold for a tridimensional one can lead to higher activity. Furthermore, even more remarkable was the comparison between the $sp^3$-drug enantiomers (Axitinib $sp^3$-analogues). The results in the three cell lines corroborated not only a different drug sensitivity among the cell lines but also a different behavior of the two enantiomers of compound **12** in biological systems. This indicates that the absolute configuration of the $sp^3$-bioisostere fragment also plays a role in determining its bioactivity and highlights the importance of developing enantioselective catalytic strategies for the preparation of these $sp^3$-building blocks. The more sensitive line was Mel202 (Figures 2B and 3B), which corresponds with uveal melanoma, a rare cancer. Compound **12** presented higher toxicities than the marketed Axitinib drug in all cell lines tested. In addition, it showed higher viability reductions than *ent*-**12** (the enantiomer of **12**). The most significant difference was observed in the Mel202 cell line, where the activity of **12** was 8-fold higher than *ent*-**12**.

The effect of commercial Axitinib and the corresponding $sp^3$-hybridized analogues on the cell cycle or cell death were further evaluated. The studies were carried out in the three cancer cell lines mentioned above, where Axitinib and its analogues presented the highest activity in MB231 (see Table 2 below and Figure 4).

**Table 2. Cell cycle and cell death studies.** MB231 studies 72 h after treatment with the two Axitinib $sp^3$-analogues (**12** and *ent*-**12**, 20 μM) and the $sp^2$-based Axitinib drug (20 μM).

| | Cell cycle assay | | | Cell death assay | | |
|---|---|---|---|---|---|---|
| | G1/G0 | S | G2/M | Early apoptosis | Late apoptosis/ necrosis | Total cell death |
| Untreated | 61.36 % | 23.95 % | 14.69 % | 0.87 % | 2.58 % | 3.45 % |
| Axitinib | 40.77 % | 35.56 % | 23.68 % | 3.16 % | 6.41 % | 9.57 % |
| Compound **12** | 44.60 % | 33.83 % | 21.57 % | 1.74 % | 4.52 % | 6.26 % |
| Compound *ent*-**12** | 27.47 % | 66.17 % | 6.36 % | 8.10 % | 11.38 % | 19.48 % |

These results corroborate the therapeutic potential of the obtained $sp^3$-bioisostere-containing drug analogues and further suggest that the different bicyclo[2.1.1]hexane enantiomers could affect the cellular pathways differently. In this regard, cells treated with compound *ent*-**12** presented a more altered cycle and cell death than **12** and Axitinib. Remarkably, *ent*-**12** was able to induce two-fold more cell death than the marketed drug Axitinib.

## Claims

1. - A compound of formula **I**:

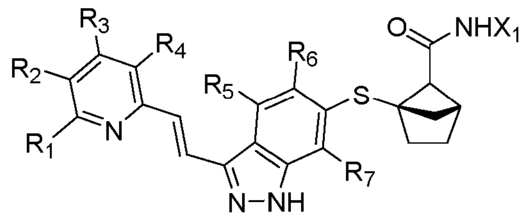

**I** ,

a stereoisomer, a pharmaceutically acceptable salt or a solvate thereof;
wherein $X_1$ is selected from H, C1-C6 alkyl, C1-C6 fluorinated alkyl and C6-C10 aryl; and
wherein groups $R_1$-$R_7$ are independently selected from H, $^2$H, halogen, C1-C6 alkyl, C1-C6 fluorinated alkyl and C6 aryl.

2. - The compound according to claim 1, wherein the C1-C6 fluorinated alkyl is a C1-C6 perfluorinated alkyl.

3. - The compound according to claim 1 or 2, wherein $X_1$ is selected from H, C1-C3 alkyl, C1-C3 perfluorinated alkyl, preferably is H, $CH_3$ or $CF_3$, more preferably is $CH_3$.

**4.** - The compound according to any one of claims 1 to 3, wherein the groups $R_1$-$R_7$ are independently selected from H, $^2H$, halogen, C1-C3 alkyl, C1-C3 perfluorinated alkyl and phenyl, preferably wherein the groups $R_1$-$R_7$ are independently selected from H, $^2H$, halogen, $CH_3$ and $CF_3$.

**5.** - The compound according to any one of the preceding claims, wherein $R_5$, $R_6$ and $R_7$ are H, preferably wherein all of $R_1$-$R_7$ are H.

**6.** - The compound according to any one of the preceding claims, wherein the compound of formula **I** is a mixture of enantiomers **Ia** and **Ib:**

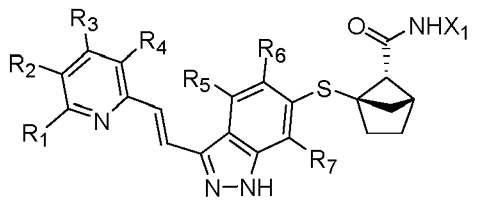

**Ia**

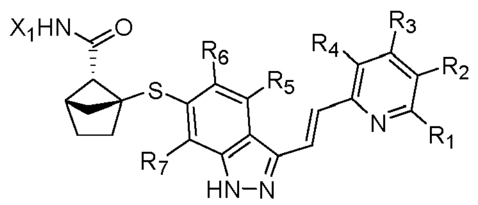

**Ib**

**7.** - The compound according to any of claims 1 to 5, wherein the compound of formula **I** is:

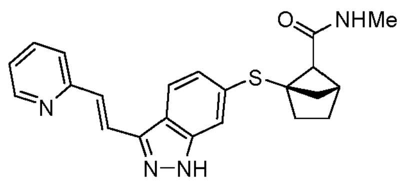

**I**

**8.** - The compound according to claim 7 which is a mixture of enantiomers **Ia'** and **Ib'**

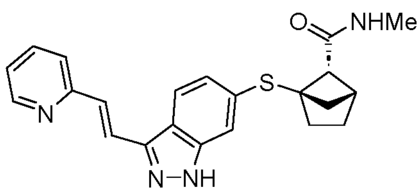

**Ia'**

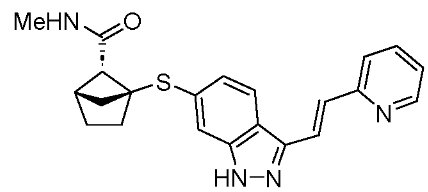

**Ib'**

**9.** - The compound according to claim 8 wherein the molar ratio between **Ia'** and **Ib'** is comprised between 99.9:0.1 and 90:10, preferably from 99:1 to 95:5, or is comprised between 10:90 and 0.1:99.9, preferably between 5:95 and 1:99.

**10.** - A pharmaceutical composition comprising the compound **I** according to any one of claim 1 to 9.

**11.** - A method for preparing a compound of formula I comprising the steps of:

i. reacting a compound of formula **XI** with a compound of formula **XII** under light irradiation and in the presence of a base:

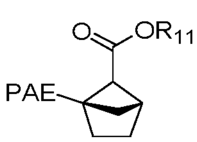

**XI** ,

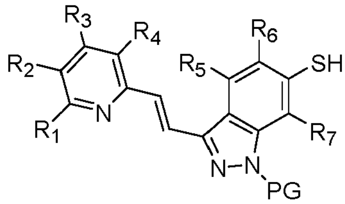

**XII** ,

to obtain compound XIII:

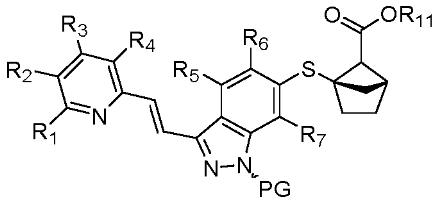

**XIII** ,

wherein PAE is a photoredox-active ester; PG is a N-protecting group; $R_{11}$ is a C1-C6 alkyl or a C6-C10 aryl; and $R_1$-$R_7$ are independently selected from H, $^2H$, halogen, C1-C6 alkyl, C1-C6 fluorinated alkyl and C6 aryl;

ii. ester hydrolysis of compound **XIII** under basic conditions to obtain free acid **XIV**

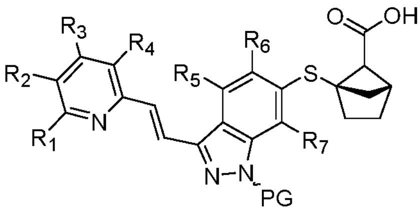

**XIV** ;

iii. reacting free acid **XIV** with an amine of formula $X_1NH_2$ in presence of an acid-activating reagent, wherein $X_1$ is selected from H, C1-C6 alkyl, C1-C6 fluorinated alkyl and C6-C10 aryl, to obtain a compound of formula **XV:**

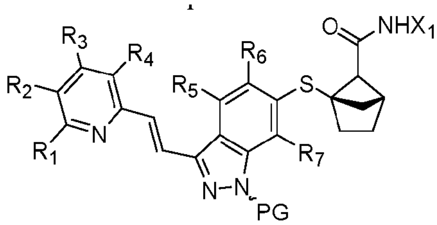

**XV** ;

iv. subjecting compound **XV** to acidic hydrolysis to remove the protecting group (PG) and thereby to obtain a compound of formula **I.**

**12.** - The method according to claim 11, comprising the steps of:

i. Reacting an alkyne of formula **II** with the organomagnesium compound of formula **III:**

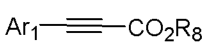

**II**

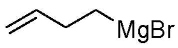

**III** ,

to obtain a compound of formula **IV:**

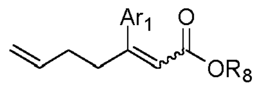

**IV** ,

wherein $Ar_1$ is a C6-C10 (hetero)aryl and Rs is a C1-C6 alkyl group or a C6-C10 aryl group;

ii. Hydrolysis under basic conditions of compound **IV** into compound **V**

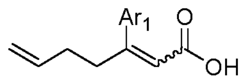

**V** ;

iii. Coupling compound **V** with compound **VI**

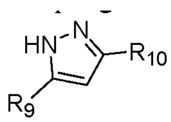

**VI** ,

to obtain compound **VII**

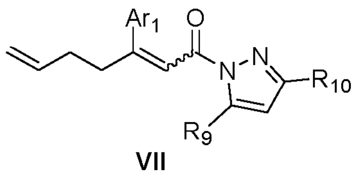

**VII**

wherein $R_9$ and $R_{10}$ are independently selected from H, C1-C6 alkyl and C6-C10 aryl;

iv. Subjecting compound **VII** to a crossed [2+2] photocycloaddition, optionally in an enantioselective manner, in presence of a rhodium catalyst, optionally an enantiomerically pure rhodium catalyst, to obtain compound **VIII:**

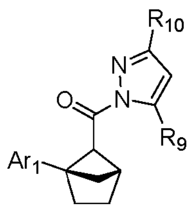

**VIII** ;

v. Subjecting compound **VIII** to esterification with an alcohol of formula $R_{11}OH$ in presence of an inorganic chloride salt and tertiary amine, wherein $R_{11}$ is a C1-C6 alkyl or a C6-C10 aryl, to obtain a compound of formula **IX:**

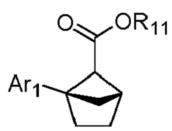

**IX** ;

vi. Oxidizing compound **IX** to compound **X** in the presence of a Ru(III) salt and an oxidant,

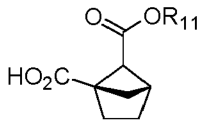

**X** ;

vii. Derivatization of compound **X** to obtain compound **XI:**

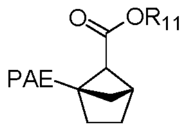

**XI**

Wherein PAE is a photoredox-active ester and $R_{11}$ is defined as above;

viii. reacting the compound of formula **XI** with a compound of formula **XII** under light irradiation and in the presence of a base:

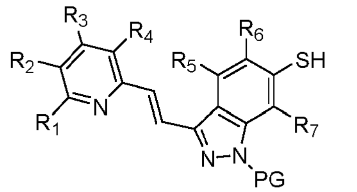

**XII** ,

to obtain compound **XIII:**

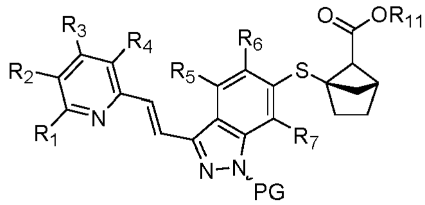

**XIII**

wherein PAE and $R_{11}$ are as defined as above; PG is a N-protecting group; and $R_1$-$R_7$ are independently selected from H, $^2H$, halogen, C1-C6 alkyl, C1-C6 fluorinated alkyl and C6 aryl;

ix. ester hydrolysis of compound **XIII** under basic conditions to obtain free acid **XIV**

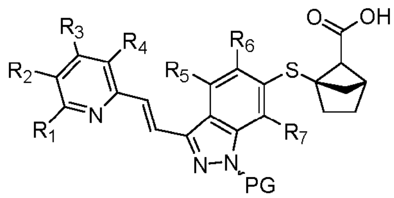

**XIV** ;

x. reacting free acid **XIV** with an amine of formula $X_1NH_2$ in presence of an acid-activating reagent, wherein $X_1$ is selected from H, C1-C6 alkyl, C1-C6 fluorinated alkyl and C6-C10 aryl, to obtain a compound of formula **XV;**

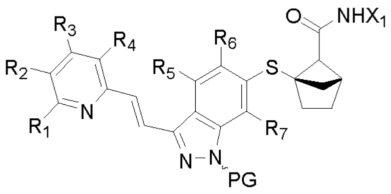

XV ;

xi. subjecting compound **XV** to acidic hydrolysis to remove the protecting group (PG) and thereby to obtain a compound of formula **I.**

**13.** - A compound of formula **I** according to any one of claims 1 to 9 or a pharmaceutical composition according to claim 10 for use in medicine.

**14.** - A compound of formula **I** according to any one of claims 1 to 9 or a pharmaceutical composition according to claim 10 for use in the treatment of cancer.

**15.** - The compound or pharmaceutical composition for use according to claim 14, wherein the cancer is selected from uveal melanoma, breast cancer and colon cancer.

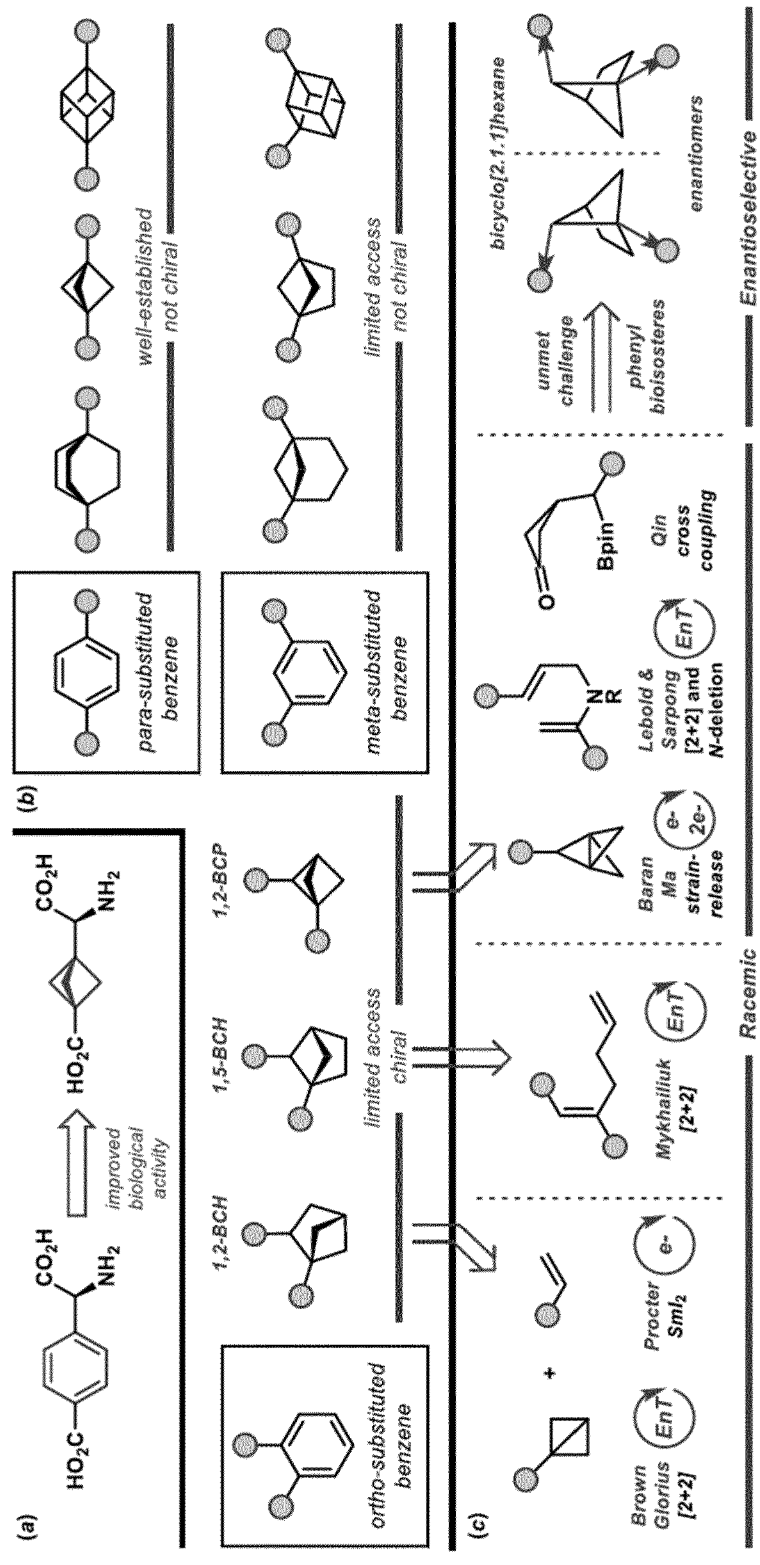
(a)
improved biological activity
(b)
para-substituted benzene
well-established not chiral
meta-substituted benzene
limited access not chiral
ortho-substituted benzene
1,2-BCH
1,5-BCH
1,2-BCP
limited access chiral
(c)
Brown Glorius [2+2]
EnT
Procter $SmI_2$
e-
Mykhailiuk [2+2]
Baran Ma strain-release
2e-
Lebold & Sarpong [2+2] and N-deletion
Qin cross coupling
unmet challenge
phenyl bioisosteres
bicyclo[2.1.1]hexane
enantiomers
Racemic
Enantioselective

Figure 1

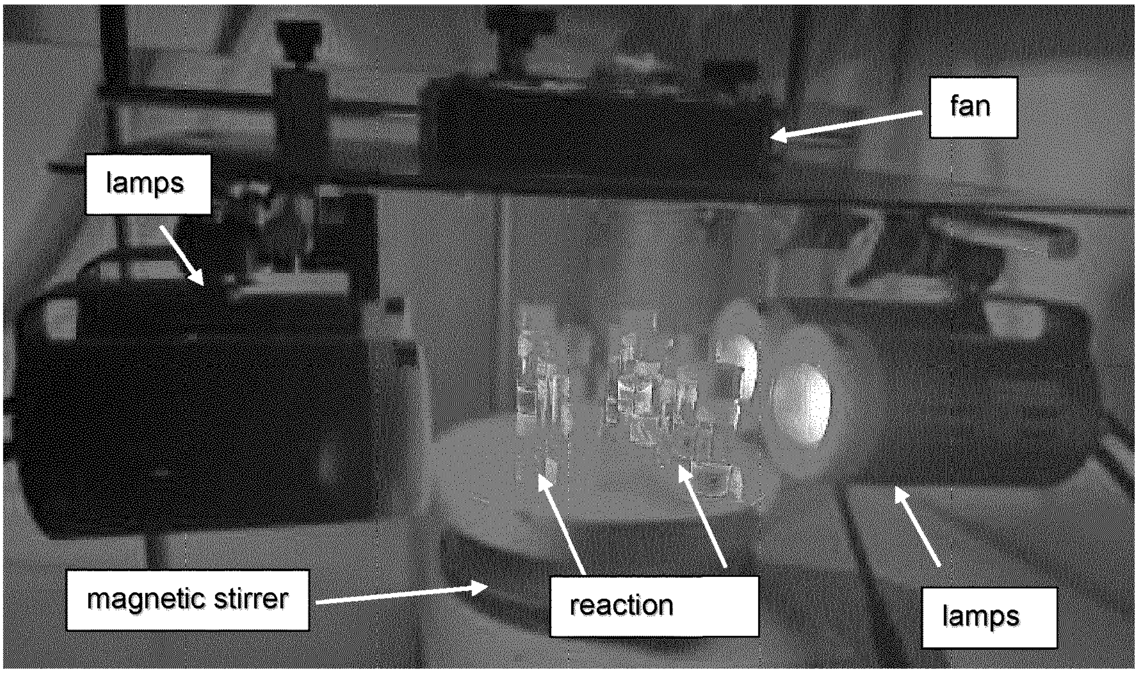


Figure 2

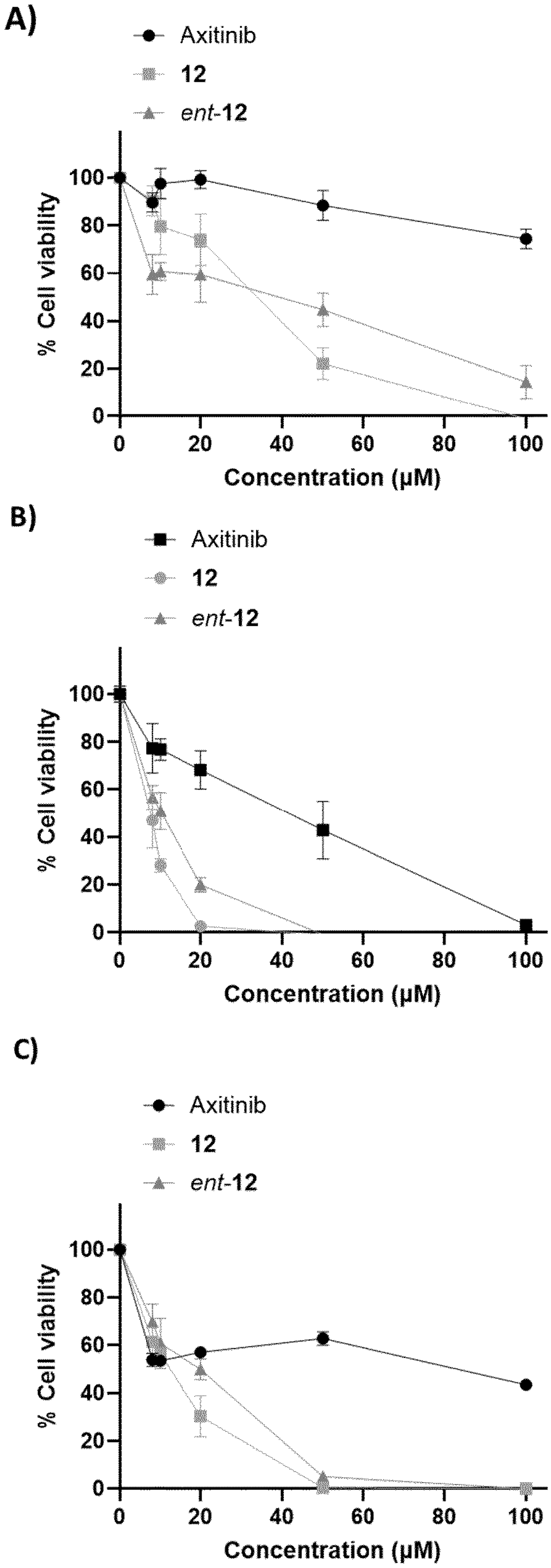
A)
Axitinib
12
ent-12
% Cell viability
100
80
60
40
20
0
0
20
40
60
80
100
Concentration (μM)
B)
Axitinib
12
ent-12
% Cell viability
100
80
60
40
20
0
0
20
40
60
80
100
Concentration (μM)
C)
Axitinib
12
ent-12
% Cell viability
100
80
60
40
20
0
0
20
40
60
80
100
Concentration (μM)

Figure 3

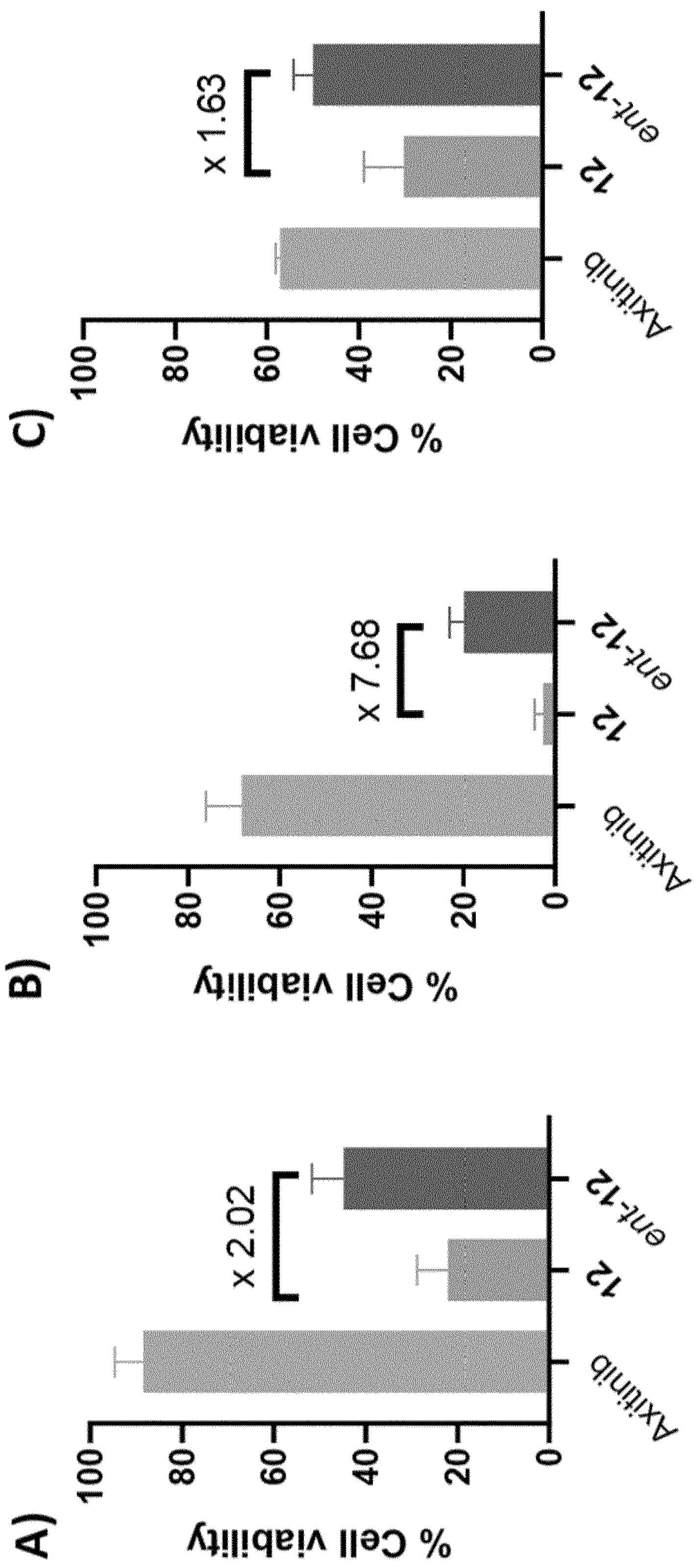
A)
% Cell viability
100
80
60
40
20
0
Axitinib
12
ent-12
x 2.02
B)
% Cell viability
100
80
60
40
20
0
Axitinib
12
ent-12
x 7.68
C)
% Cell viability
100
80
60
40
20
0
Axitinib
12
ent-12
x 1.63

Figure 4

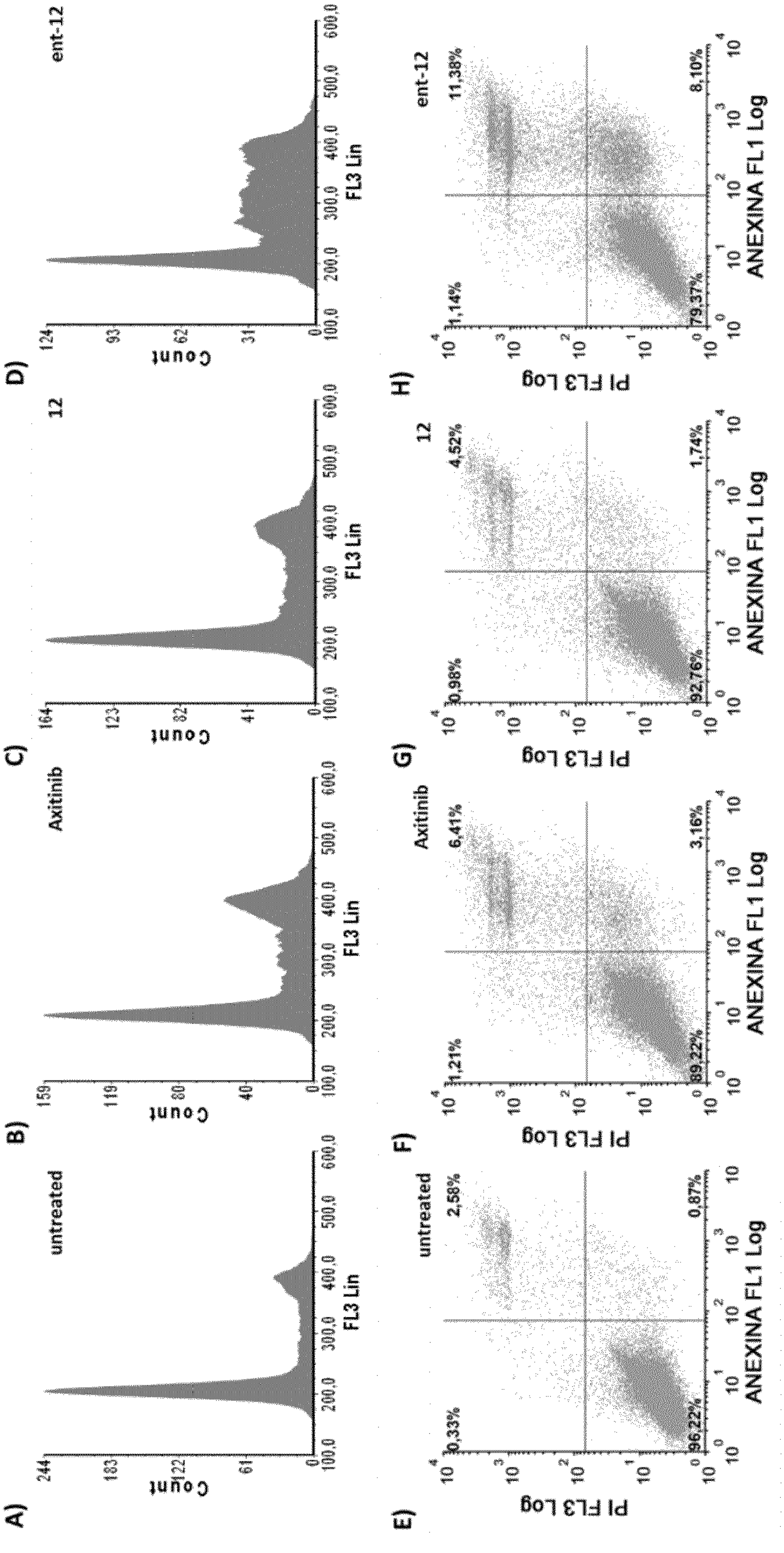
A)
untreated
B)
Axitinib
C)
12
D)
ent-12
Count
FL3 Lin
E)
untreated
0,33%
2,58%
96,22%
0,87%
F)
Axitinib
1,21%
6,41%
89,22%
3,16%
G)
12
0,98%
4,52%
92,76%
1,74%
H)
ent-12
1,14%
11,38%
79,37%
8,10%
PI FL3 Log
ANEXINA FL1 Log

Figure 5

Europäisches Patentamt
European Patent Office
Office européen des brevets

# EUROPEAN SEARCH REPORT

Application Number
EP 24 38 2968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ZHAO JIN-XIN ET AL: "1,2-Difunctionalized bicyclo[1.1.1]pentanes: Long-sought-after mimetics for ortho / meta -substituted arenes", PROCEEDINGS OF THE NATIONAL ACADEMY OF SCIENCES (PNAS), vol. 118, no. 28, 8 July 2021 (2021-07-08), XP093243720, ISSN: 0027-8424, DOI: 10.1073/pnas.2108881118 * figure 4; compounds (+)-20, (-)-20 * ----- | 1-6, 10-15 | INV. C07D401/06 A61P35/00 A61K31/4439 |
| A | WO 01/02369 A2 (AGOURON PHARMA [US]) 11 January 2001 (2001-01-11) * page 340; claims 1, 10, 12, 13, 15; example 59(f) * ----- | 1-15 | |

TECHNICAL FIELDS SEARCHED (IPC)

C07D
A61P
A61K

The present search report has been drawn up for all claims

| Place of search | Date of completion of the search | Examiner |
|---|---|---|
| Munich | 28 January 2025 | Moriggi, J |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 38 2968

This annex lists the patent family members relating to the patent documents cited in the above-mentioned European search report. The members are as contained in the European Patent Office EDP file on
The European Patent Office is in no way liable for these particulars which are merely given for the purpose of information.

28-01-2025

| Patent document cited in search report | Publication date | Patent family member(s) | Publication date |
|---|---|---|---|
| WO 0102369 A2 | 11-01-2001 | AP 1486 A | 01-11-2005 |
| | | AR 035554 A1 | 16-06-2004 |
| | | AR 065937 A2 | 15-07-2009 |
| | | AT E376543 T1 | 15-11-2007 |
| | | AU 777701 B2 | 28-10-2004 |
| | | BE 2013C015 I2 | 21-05-2019 |
| | | BG 66070 B1 | 31-01-2011 |
| | | BR 0012352 A | 14-05-2002 |
| | | CA 2383630 A1 | 11-01-2001 |
| | | CN 1374950 A | 16-10-2002 |
| | | CN 1495171 A | 12-05-2004 |
| | | CO 5190686 A1 | 29-08-2002 |
| | | CR 6517 A | 21-09-2006 |
| | | CR 10194 A | 30-09-2008 |
| | | CY 1107147 T1 | 24-10-2012 |
| | | CY 1107148 T1 | 25-01-2012 |
| | | CY 2013009 I1 | 13-04-2016 |
| | | CZ 20014634 A3 | 11-09-2002 |
| | | DE 60036879 T2 | 14-02-2008 |
| | | DE 60037211 T2 | 11-12-2008 |
| | | DK 1218348 T3 | 25-02-2008 |
| | | DK 1614683 T3 | 25-03-2008 |
| | | DZ 3191 A1 | 11-01-2001 |
| | | EA 200200120 A1 | 29-08-2002 |
| | | EE 200100717 A | 17-02-2003 |
| | | EP 1218348 A2 | 03-07-2002 |
| | | EP 1614683 A1 | 11-01-2006 |
| | | ES 2293906 T3 | 01-04-2008 |
| | | ES 2296014 T3 | 16-04-2008 |
| | | FR 13C0010 I1 | 22-03-2013 |
| | | GE P20063885 B | 10-08-2006 |
| | | GT 200000107 A | 21-12-2001 |
| | | HK 1048813 A1 | 17-04-2003 |
| | | HK 1065037 A1 | 08-02-2005 |
| | | HK 1085470 A1 | 25-08-2006 |
| | | HR P20020109 A2 | 31-12-2003 |
| | | HU 228502 B1 | 28-03-2013 |
| | | IS 6207 A | 19-12-2001 |
| | | JO 2319 B1 | 12-09-2005 |
| | | JP 3878849 B2 | 07-02-2007 |
| | | JP 3969669 B2 | 05-09-2007 |
| | | JP 2003503481 A | 28-01-2003 |
| | | JP 2006348043 A | 28-12-2006 |
| | | KR 20020027379 A | 13-04-2002 |
| | | LU 92154 I2 | 22-04-2013 |
| | | MA 26803 A1 | 20-12-2004 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 24 38 2968

This annex lists the patent family members relating to the patent documents cited in the above-mentioned European search report. The members are as contained in the European Patent Office EDP file on
The European Patent Office is in no way liable for these particulars which are merely given for the purpose of information.

28-01-2025

| Patent document cited in search report | Publication date | Patent family member(s) | Publication date |
|---|---|---|---|
| | | ME 00385 B | 10-05-2011 |
| | | MX PA01012795 A | 02-09-2002 |
| | | MY 137622 A | 27-02-2009 |
| | | MY 139999 A | 30-11-2009 |
| | | NO 322507 B1 | 16-10-2006 |
| | | NO 2013004 I1 | 18-03-2013 |
| | | NZ 516676 A | 26-09-2003 |
| | | OA 11980 A | 18-04-2006 |
| | | PA 8498001 A1 | 26-08-2002 |
| | | PE 20010306 A1 | 29-03-2001 |
| | | PL 355757 A1 | 17-05-2004 |
| | | PT 1218348 E | 14-12-2007 |
| | | PT 1614683 E | 24-01-2008 |
| | | SI 1218348 T1 | 29-02-2008 |
| | | SI 1614683 T1 | 29-02-2008 |
| | | SK 19252001 A3 | 06-11-2002 |
| | | SV 2002000121 A | 02-12-2002 |
| | | TN SN00146 A1 | 10-11-2005 |
| | | UA 66933 C2 | 15-06-2004 |
| | | UY 26231 A1 | 31-01-2001 |
| | | WO 0102369 A2 | 11-01-2001 |
| | | YU 92901 A | 03-09-2004 |
| | | ZA 200110061 B | 06-02-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Med. Chem.*, 1996, vol. 39, 2874-2876
- **DENISENKO A et al.** *Angew. Chem. Int. Ed.*, 2020, vol. 59, 20515-20521
- **PABLO GARRIDO-GARCIA et al.** *ChemRxiv*, 2023
- **NA WEI et al.** *Molecules*, 2018, vol. 23 (4), 747
- **JIN-XIN ZHAO et al.** *Proc. Natl. Acad. Sci. USA.*, 2021, vol. 118 (28), e2108881118
- **L.-H. ZHAI et al.** *Org. Process Res. Dev.*, 2015, vol. 19, 849-857
- **P. R. KYM et al.** *J. Med. Chem.*, 2006, vol. 49 (7), 2339-2352
- **B.-L. LI et al.** *Chinese Chemical Letters*, 2014, vol. 25, 989-994
- **R. A. BARTSCH et al.** *J. Heterocycl. Chem.*, 1984, vol. 21, 1063-1064
- **J. YU et al.** *Beilstein J. Org. Chem.*, 2018, vol. 14, 786-795
- **PABLO GARRIDO-GARCÍA et al.** *ChemRxiv*, 2023
- **ERIC MEGGERS**. *Nat. Protoc.*, 2018, vol. 13, 605-632
- **J. MA et al.** *Nat. Protoc.*, 2018, vol. 13, 605-632
- **P. GARRIDO-GARCIA et al.** *ChemRxiv*, 2023
- **W. H. BROOKS et al.** *Curr. Top. Med. Chem.*, 2011, vol. 11, 760-770